# FASCICULE DE BREVET EUROPEEN

(11) **EP 1 458 721 B1**
(45) Date de publication et mention de la délivrance du brevet: **22.11.2006**
(21) Numéro de dépôt: 02796852.8
(22) Date de dépôt: 20.11.2002
(51) Int. Cl.: C07D 487/04, A61K 31/50, C07D 237/00, C07D 209/00

(54) **DERIVES DE 3-HETEROARYL-3,5-DIHYDRO-4-OXO-4H-PYRIDAZINO [4,5-B] INDOLE-1-CARBOXAMIDE, LEUR PREPARATION ET LEUR APPLICATION EN THERAPEUTIQUE**
3-HETEROARYL-3,5-DIHYDRO-4-OXO-4H-PYRIDAZINO[4,5-B]INDOL-1-KARBOXAMID-DERIVATE, IHRE HERSTELLUNG UND THERAPEUTISCHE VERWENDUNG
3-HETEROARYL-3,5-DIHYDRO-4-OXO-4H-PYRIDAZINO [4,5-B]INDOLE-1-CARBOXAMIDE DERIVATIVES, THEIR PREPARATION AND THERAPEUTIC USE

(30) Priorité: 21.12.2001 FR 0116701
(43) Date de publication de la demande: 22.09.2004
(73) Titulaire: Sanofi-Aventis, 75013 Paris (FR)
(72) Inventeur: BURNIER, Philippe, F-78600 Maisons-Laffitte (FR); FROISSANT, Jacques, F-41160 Brevainville (FR); MARABOUT, Benoît, F-91300 Massy (FR); MARGUET, Frank, F-91370 Verrières-Le-Buisson (FR); PUECH, Frédéric, F-78170 La Celle Saint Cloud (FR)
(74) Mandataire: Ludwig, Jacques
(86) Numéro de dépôt international: PCT/FR2002/003979
(87) Numéro de publication internationale: WO 2003/055884

(56) Documents cités:
- WO-A-00/44751
- WO-A-02/08229
- WO-A-98/15552

## Description

L'invention a pour objet des composés dérivés de 3-hétéroaryl-3,5-dihydro-4-oxo-4*H*-pyridazino[4,5-*b*]indole-1-carboxamide.

On connaît déjà des composés dérivés de 3,5-dihydropyridazino[4,5-*b*]indole, décrits dans le document WO-A-0044751, affins in vitro pour les récepteurs de type périphérique aux benzodiazépines (sites p ou PBR).
Il existe toujours une nécessité de trouver et de développer des produits présentant une bonne activité in vivo.
L'invention répond à ce but en proposant des composés nouveaux, qui présentent une affinité in vitro et in vivo pour les récepteurs de type périphérique aux benzodiazépines.

Un premier objet de l'invention concerne les composés répondant à la formule générale (I) ci-après.
Un autre objet de l'invention concerne des procédés de préparation des composés de formule générale (I).
Un autre objet de l'invention concerne des composés utilisables notamment en tant qu'intermédiaires de synthèse des composés de formule générale (I).
Un autre objet de l'invention concerne les utilisations des composés de formule générale (I) notamment dans des médicaments ou dans des compositions pharmaceutiques.

Les composés de l'invention répondent à la formule générale (I) : 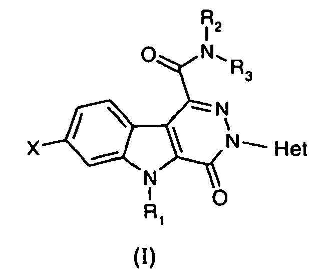 dans laquelle
X représente un atome d'hydrogène ou d'halogène,
R₁ représente un atome d'hydrogène ou un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle, morpholinyle ou 4-alkylpipérazinyle, et
Het représente un groupe hétéroaromatique de type pyridinyle, 1-oxydopyridinyle, quinolinyle, isoquinolinyle, pyrimidinyle, pyrazinyle, pyridazinyle, le groupe hétéroaromatique pouvant porter un ou plusieurs atomes d'halogène et/ou un ou plusieurs groupes (C₁-C₄)alkyle, (C₁-C₄)alcoxyle.

Dans le cadre de la présente invention :
- un groupe (C₁-C₄)alkyle représente un groupe aliphatique, comportant de 1 à 4 atomes de carbone, saturé linéaire ou ramifié. A titre d'exemples, on peut citer les groupes méthyle, éthyle, propyle, isopropyle, butyle, isobutyle, terbutyle.
- Un groupe (C₁-C₄)alcoxyle représente un radical oxygène substitué par un groupe alkyle, comportant de 1 à 4 atomes de carbone, tel que défini précédemment.
Des composés préférés selon l'invention sont les composés pour lesquels
X représente un atome d'halogène ; et/ou
R₁ représente un (C₁-C₄)alkyle ; et/ou R₂ et R₃ représentent chacun, indépendamment l'un de l'autre, un atome d'hydrogène, un groupe (C₁-C₄)alkyle, plus particulièrement un méthyle ou un éthyle, ou bien R₂ et R₃ forment, avec l'atome d'azote qui les porte, un groupe pyrrolidinyle, pipéridinyle, morpholinyle ou 4-alkylpipérazinyle ; et/ou
Het représente un groupe hétéroaromatique de type pyridinyle, quinolinyle, isoquinolinyle, pyrimidinyle, pyrazinyle, pyridazinyle, pouvant porter un ou plusieurs atomes d'halogène, plus particulièrement un atome de brome, et/ou un ou plusieurs groupes (C₁-C₄)alkyle, plus particulièrement un méthyle, (C₁-C₄)alcoxyle, plus particulièrement un méthoxyle.

Des composés pour lesquels à la fois X, R₁, R₂, R₃ et Het sont tels que définis ci-dessus dans les sous-groupes de composés préférés, sont particulièrement préférés et plus spécifiquement parmi ceux-ci les composés pour lesquels :
X représente un atome de chlore, R₁ représente un groupe méthyle.

A titre d'exemple, des composés de l'invention sont les suivants :
**1** : 7-fluoro-*N,N*,5-triméthyl-4-oxo-3-(pyridin-2-yl)-3,5-dihydro-4*H*-pyridazino[4,5-*b*]indole-1-carboxamide
**2 :** 7-fluoro-*N,N*,5-triméthyl-4-oxo-3-(pyridin-3-yl)-3,5-dihydro-4*H*-pyridazino[4,5-*b*]indole-1-carboxamide
**3 :** chlorhydrate de 7-fluoro-*N,N*,5-triméthyl-4-oxo-3-(pyridin-4-yl)-3,5-dihydro-4*H-*pyridazino[4,5-*b*]indole-1-carboxamide (1 :1)
**4 :** 7-fluoro-*N,N*,5-triméthyl-4-oxo-3-(2-méthoxypyridin-5-yl)-3,5-dihydro-4*H-*pyridazino[4,5-*b*]indole-1-carboxamide
**5 :** 7-fluoro-*N,N*,5-triméthyl-4-oxo-3-(quinolin-3-yl)-3,5-dihydro-4*H*-pyridazino[4,5-b]indole-1-carboxamide
**6 :** 1-[[7-fluoro-5-méthyl-3-(pyrimidin-2-yl)-4-oxo-3,5-dihydro-4*H*-pyridazino[4,5-*b*]indol-1-yl]carbonyl]pyrrolidine
**7 :** chlorhydrate de 4-méthyl-1-[[7-fluoro-5-méthyl-3-(pyridin-3-yl)-4-oxo-3,5-dihydro-4*H*-pyridazino[4,5-*b*]indol-1-yl]carbonyl]pipérazine (1:1)
**8 :** 1-[[7-fluoro-5-méthyl-3-(pyridin-3-yl)-4-oxo-3,5-dihydro-4*H*-pyridazino[4,5-*b*]indol-1-yl]carbonyl]pyrrolidine
**9 :** 7-fluoro-*N*,5-diméthyl-4-oxo-3-(pyridin-3-yl)-3,5-dihydro-4*H*-pyridazino[4,5-*b*]indole-1-carboxamide
**10 :** 7-fluoro-5-méthyl-4-oxo-3-(pyridin-3-yl)-3,5-dihydro-4*H*-pyridazino[4,5-*b*]indole-1-carboxamide
**11 :** 7-chloro-*N,N*,5-triméthyl-4-oxo-3-(pyridin-2-yl)-3,5-dihydro-4*H*-pyridazino[4,5-*b*]indole-1-carboxamide
**12 :** chlorhydrate de 7-chloro-*N,N*,5-triméthyl-4-oxo-3-(pyridin-3-yl)-3,5-dihydro-4*H-*pyridazino[4,5-*b*]indole-1-carboxamide (1 :1)
**13 :** 7-chloro-*N,N*,5-triméthyl-4-oxo-3-(pyridin-4-yl)-3,5-dihydro-4*H*-pyridazino[4,5-*b*]indole-1-carboxamide
**14 :** chlorhydrate de 7-chloro-*N,N*,5-triméthyl-4-oxo-3-(pyridin-4-yl)-3,5-dihydro-4*H-*pyridazino[4,5-*b*]indole-1-carboxamide (1 :1)
**15 :** 7-chloro-*N,N*,5-triméthyl-4-oxo-3-(5-méthylpyridin-2-yl)-3,5-dihydro-4*H-*pyridazino[4,5-*b*]indole-1-carboxamide
**16 :** 7-chloro-*N*,*N*,5-triméthyl-4-oxo-3-(2-méthoxypyridin-5-yl)-3,5-dihydro-4*H-*pyridazino[4,5-*b*]indole-1-carboxamide
**17 :** 7-chloro-*N,N*,5-triméthyl-4-oxo-3-(2-méthylpyridin-5-yl)-3,5-dihydro-4*H-*pyridazino[4,5-*b*]indole-1-carboxamide
**18 :** 7-chloro-*N*,*N*,5-triméthyl-4-oxo-3-(2-bromopyridin-5-yl)-3,5-dihydro-4*H-*pyridazino[4,5-*b*]indole-1-carboxamide
**19 :** 7-chloro-*N,N*,5-triméthyl-4-oxo-3-(quinolin-3-yl)-3,5-dihydro-4*H*-pyridazino[4,5-*b*]indole-1-carboxamide
**20 :** 7-chloro-*N,N*,5-triméthyl-4-oxo-3-(isoquinolin-4-yl)-3,5-dihydro-4*H-*pyridazino[4,5-*b*]indole-1-carboxamide
**21 :** 7-chloro-*N,N*,5-triméthyl-4-oxo-3-(6-méthylpyridazin-3-yl)-3,5-dihydro-4*H-*pyridazino[4,5-*b*]indole-1-carboxamide
**22 :** 7-chloro-*N,N*,5-triméthyl-4-oxo-3-(pyrimidin-5-yl)-3,5-dihydro-4*H-*pyridazino[4,5-*b*]indole-1-carboxamide
**23 :** 7-chloro-*N,N*,5-triméthyl-4-oxo-3-(pyrimidin-2-yl)-3,5-dihydro-4*H-*pyridazino[4,5-*b*]indole-1-carboxamide
**24 :** 7-chloro-*N,N*,5-triméthyl-4-oxo-3-(pyrazin-2-yl)-3,5-dihydro-4*H*-pyridazino[4,5-*b*]indole-1-carboxamide
**25 :** chlorhydrate de 1-[[7-chloro-5-méthyl-3-(pyridin-3-yl)-4-oxo-3,5-dihydro-4*H-*pyridazino[4,5-*b*]indol-1-yl]carbonyl]pyrrolidine (1:1)
**26 :** chlorhydrate de 4-méthyl-1-[[7-chloro-5-méthyl-3-(pyridin-3-yl)-4-oxo-3,5-dihydro-4*H*-pyridazino[4,5-*b*]indol-1-yl]carbonyl]pipérazine (1:1)
**27 :** 1-[[7-chloro-5-méthyl-3-(pyridin-4-yl)-4-oxo-3,5-dihydro-4*H*-pyridazino[4,5-*b*]indol-1-yl]carbonyl]pyrrolidine
**28 :** 7-chloro-*N*,5-diméthyl-4-oxo-3-(pyridin-4-yl)-3,5-dihydro-4*H*-pyridazino[4,5-*b*]indole-1-carboxamide
**29 :** 7-chloro-5-méthyl-4-oxo-3-(pyridin-4-yl)-3,5-dihydro-4*H*-pyridazino[4,5-*b*]indole-1-carboxamide
**30 :** 7-chloro-*N*,*N*,5-triméthyl-4-oxo-3-(4-méthoxypyridin-2-yl)-3,5-dihydro-4*H-*pyridazino[4,5-*b*]indole-1-carboxamide
**31 :** 1-[[7-chloro-5-méthyl-3-(pyridin-4-yl)-4-oxo-3,5-dihydro-4*H*-pyridazino[4,5-*b*]indol-1-yl]carbonyl]morpholine
**32 :** 7-chloro-*N*,*N*-diéthyl-5-méthyl-4-oxo-3-(pyridin-4-yl)-3,5-dihydro-4*H-*pyridazino[4,5-*b*]indole-1-carboxamide
**33 :** 7-chloro-*N*-éthyl-*N*,5-diméthyl-4-oxo-3-(pyridin-4-yl)-3,5-dihydro-4*H-*pyridazino[4,5-*b*]indole-1-carboxamide
**34 :** 1-[[7-chloro-5-méthyl-3-(pyridin-4-yl)-4-oxo-3,5-dihydro-4*H*-pyridazino[4,5-*b*]indol-1-yl]carbonyl]pipéridine
**35 :** 7-chloro-*N,N*,5-triméthyl-4-oxo-3-(2-méthylpyridin-4-yl)-3,5-dihydro-4*H-*pyridazino[4,5-*b*]indole-1-carboxamide
**36 :** 1-[[7-chloro-5-méthyl-3-(2-méthylpyridin-4-yl)-4-oxo-3,5-dihydro-4*H-*pyridazino[4,5-*b*]indol-1-yl]carbonyl]pyrrolidine
**37 :** 7-chloro-*N,N*,5-triméthyl-3-(1-oxydopyridin-4-yl)-4-oxo-3,5-dihydro-4*H-*pyridazino[4,5-*b*]indole-1-carboxamide
**38 :** 7-chloro-3-(2-méthoxypyridin-4-yl)-*N*,*N*,5-triméthyl-4-oxo-3,5-dihydro-4*H-*pyridazino[4,5-*b*]indole-1-carboxamide
**39 :** 3-(2-bromopyridin-4-yl)-7-chloro-*N,N*,5-triméthyl-4-oxo-3,5-dihydro-4*H-*pyridazino[4,5-*b*]indole-1-carboxamide

Les composés de l'invention peuvent exister à l'état de bases, de sels d'addition à des acides, de solvats ou d'hydrates.

Les composés de formule générale (I) peuvent être préparés par les procédés illustrés dans ce qui suit.

Dans toute la suite de la description, les composés intermédiaires (II), (III), (IV), (V) sont ceux présentés dans le schéma ci-après.

Selon une première voie de préparation, un composé de formule générale (II), dans laquelle X et R₁ sont tels que définis ci-dessus et R' et R" représentent chacun, indépendamment l'un de l'autre, un groupe (C₁-C₄)alkyle, est traité avec une hétéroarylhydrazine dans un solvant polaire en présence d'acide, à la température de reflux, pour obtenir un ester de formule générale (III) dans laquelle X, R₁, Het et R" sont tels que définis ci-dessus.
Cet ester est transformé en amide de formule générale (I) par action d'une amine de formule générale HNR₂R₃, dans laquelle R₂ et R₃ sont tels que définis ci-dessus, par exemple en présence d'un dérivé trialkylaluminium dans un solvant tel que le toluène, ou bien par saponification de l'ester de formule générale (III) en acide en utilisant par exemple l'hydroxyde de lithium, dans un mélange de méthanol, d'eau et d'un solvant éthéré, puis en couplant l'acide obtenu, selon des méthodes connues de l'homme du métier, avec une amine de formule générale HNR₂R₃, telle que définie ci-dessus.

Selon une deuxième voie de préparation, un diester de formule générale (II) est traité avec de l'hydrazine en chauffant dans un solvant tel que l'acide acétique ou le toluène en présence d'acide pour obtenir un ester de formule générale (IV) dans laquelle X, R₁ et R" sont tels que définis ci-dessus.

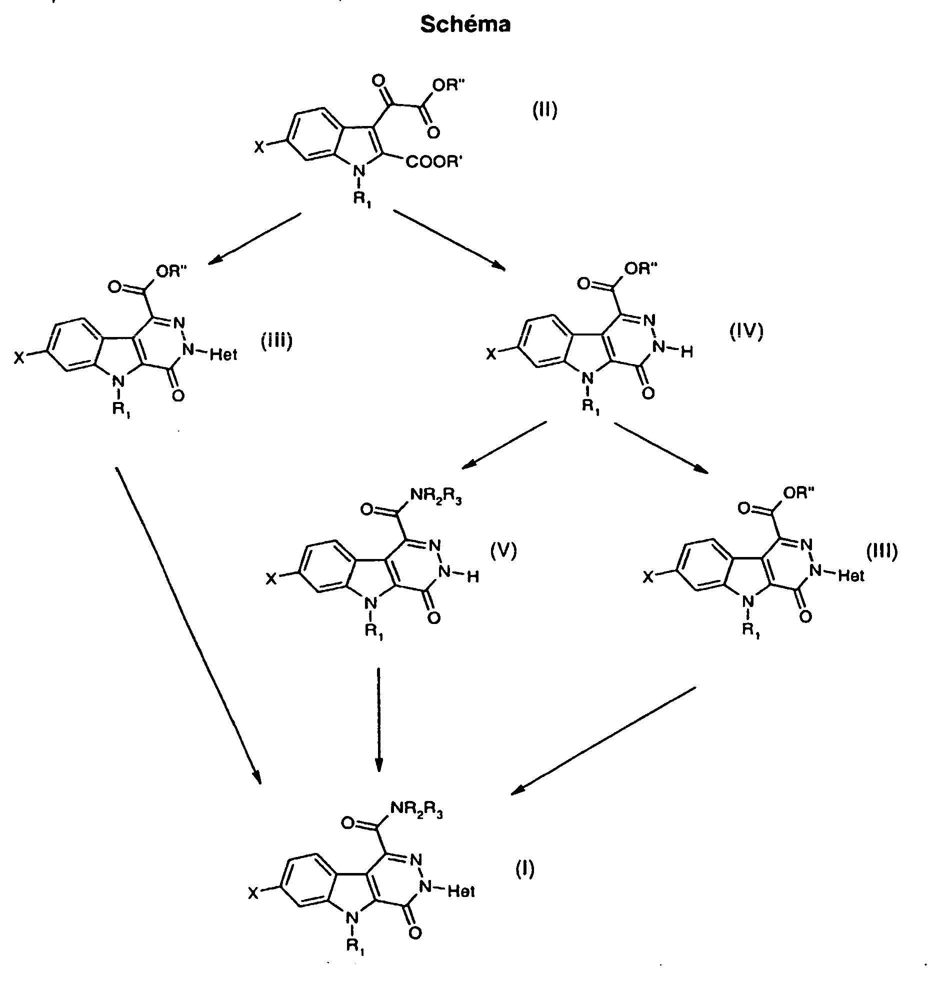

Cet ester est transformé en amide de formule générale (V), dans laquelle X, R₁, R₂ et R₃ sont tels que définis ci-dessus, par action d'une amine de formule générale HNR₂R₃, dans laquelle R₂ et R₃ sont tels que définis ci-dessus, par exemple en présence d'un dérivé trialkylaluminium dans un solvant tel que le toluène.

Finalement une *N*-hétéroarylation est effectuée par réaction de couplage en présence d'un halogénure d'hétéroaryle, ou bien d'un dérivé d'acide hétéroaryl-boronique et d'un sel métallique tel qu'un sel de cuivre conduisant à un composé de formule générale (I).
Cette réaction de *N*-hétéroarylation peut également être effectuée sur le composé de formule générale (IV) pour conduire à l'ester de formule générale (III). Cet ester est finalement transformé en amide de formule générale (I) par action d'une amine de formule générale HNR₂R₃, dans laquelle R₂ et R₃ sont tels que définis ci-dessus, par exemple, dans un mélange de solvants tel que notamment du dichlorométhane et du méthanol.
Les amides de formules générales (V) et (I) peuvent être également obtenues par saponification des esters de formules générales respectives (IV) et (III) en acides, puis en couplant les acides obtenus, avec une amine de formule générale HNR₂R₃, telle que définie ci-dessus, selon des méthodes connues de l'homme du métier.

Les dérivés d'acides boroniques portant un groupe hétéroaromatique sont disponibles dans le commerce ou peuvent être préparés par des méthodes analogues à celles connues dans la littérature (*Synth. Commun*. **1996,** 26, 3543 et

Li, et. al. *J. Med. Chem.* **1995,** 38, 4570).

Les composés de formule générale (I) pour lesquels X, R₁, R₂ et R₃ sont tels que définis précédemment et pour lesquels Het représente un groupe hétéroaromatique de type 1-oxydopyridinyle peuvent être préparés par oxydation du dérivé équivalent pour lequel Het représente un groupe hétéroaromatique de type pyridinyle, au moyen d'un agent oxydant tel que du peroxyde d'hydrogène.
Les composés de formule générale (I) pour lesquels X, R₁, R₂ et R₃ sont tels que définis précédemment et pour lesquels Het représente un groupe hétéroaromatique de type 2-halogénopyridinyle peuvent être préparés par halogénation du dérivé équivalent pour lequel Het représente un groupe hétéroaromatique de type 1-oxydopyridinyle, au moyen d'un trihalogénure de phosphore par exemple.
Les composés de formule générale (I) pour lesquels X, R₁, R₂ et R₃ sont tels que définis précédemment et pour lesquels Het représente un groupe hétéroaromatique de type 2-alcoxypyridinyle peuvent être préparés par substitution du dérivé équivalent pour lequel Het représente un groupe hétéroaromatique de type 2-halogénopyridinyle, au moyen d'un alcoolate de sodium par exemple.

La préparation des composés de départ de formule générale (II) est décrite dans le document WO-A-0044751 dans le cas où X est un atome de chlore. Cette méthode de préparation est employée de façon analogue quand X est un atome de fluor.

Un autre objet de l'invention concerne les composés de formule générale (II) 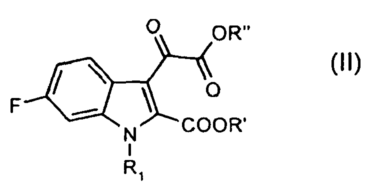 dans laquelle
R₁ représente un atome d'hydrogène ou un groupe (C₁-C₄)alkyle,
R' et R" représentent chacun, indépendamment l'un de l'autre, un groupe (Ci-C₄)alkyle,
utiles en tant qu'intermédiaires de synthèse pour la préparation des composés de formule générale (I).

Un autre objet de l'invention concerne les composés de formule générale (III) 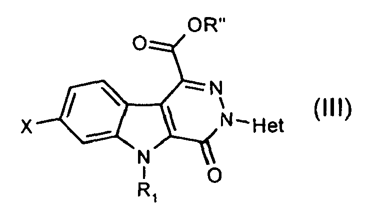 dans laquelle
X représente un atome d'hydrogène ou d'halogène,
R₁ représente un atome d'hydrogène ou un groupe (C₁-C₄)alkyle,
Het représente un groupe hétéroaromatique de type pyridinyle, 1-oxydopyridinyle, quinolinyle, isoquinolinyle, pyrimidinyle, pyrazinyle, pyridazinyle, le groupe hétéroaromatique pouvant porter un ou plusieurs atomes d'halogène et/ou un ou plusieurs groupes (C₁-C₄)alkyle, (C₁-C₄)alcoxyle,
R" représente un groupe (C₁-C₄)alkyle,
utiles en tant qu'intermédiaires de synthèse pour la préparation des composés de formule générale (I).

Un autre objet de l'invention concerne les composés de formule générale (IV) 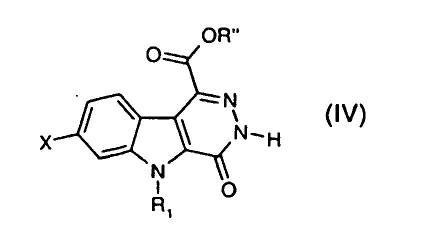 dans laquelle
X représente un atome d'hydrogène ou d'halogène,
R₁ représente un atome d'hydrogène ou un groupe (C₁-C₄)alkyle,
R" représente un groupe (C₁-C₄)alkyle,
utiles en tant qu'intermédiaires de synthèse pour la préparation des composés de formule générale (I).

Un autre objet de l'invention concerne les composés de formule générale (V) 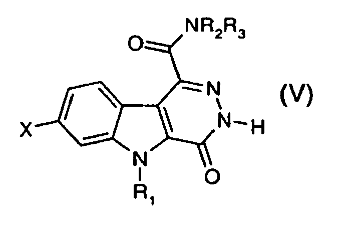 dans laquelle
X représente un atome d'hydrogène ou d'halogène,
R₁ représente un atome d'hydrogène ou un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle, morpholinyle ou 4-alkylpipérazinyle,
utiles en tant qu'intermédiaires de synthèse pour la préparation des composés de formule générale (I).

D'autres composés sont nouveaux et utiles en tant qu'intermédiaires de synthèse pour la préparation des composés de formule générale (I). Il s'agit des composés de formule générale (III) et (IV) ci-dessus, dans lesquelles R" ne représente plus un groupe (C₁-C₄)alkyle mais un atome d'hydrogène.

Les exemples qui suivent illustrent la préparation de quelques composés de l'invention. Les microanalyses élémentaires, et les spectres I.R. et R.M.N. confirment les structures des composés obtenus.

### Exemple 1 (Composé N°1)

### 7-fluoro-N,N,5-triméthyl-4-oxo-3-(pyridin-2-yl)-3,5-dihydro-4H-pyridazino[4,5-b]indole-1-carboxamide

### 1.1. 2-(4-fluoro-2-nitrophényl)-1-méthoxycarbonyléthènolate de potassium

47 g (0,419 mol) de *t*-butylate de potassium sont introduits dans 900 ml de tétrahydrofurane. Le milieu réactionnel est refroidi vers -5°C et 90 ml de méthanol sont ajoutés. 61,2 g (0,419 mol) d'oxalate d'éthyle sont ensuite introduits. Puis une solution de 54 g (0,348 mol) de 4-fluoro-2-nitrotoluène dans 100 ml de tétrahydrofurane est ajoutée goutte à goutte à basse température. L'agitation est maintenue pendant 12 h à température ambiante. La solution est filtrée. Le solide obtenu est lavé avec de l'éther diéthylique et séché sous pression réduite.
78 g d'un solide violet de 2-(4-fluoro-2-nitrophényl)-1-méthoxycarbonyléthènolate de potassium sont obtenus contenant de 10 à 20 % de 2-(4-fluoro-2-nitrophényl)-1-éthoxycarbonyléthènolate de potassium.

### 1.2. 6-fluoro-1H-indole-2-carboxylate de méthyle

Un mélange de 35 g de sel de potassium, obtenu à l'étape 1.1., dans 500 ml d'éthanol est refroidit vers 0°C. 80 ml d'acide chlorhydrique concentré sont ajoutés par petites portions. 35 g (627 mmol) de fer en poudre sont également ajoutés par portions. Le mélange est chauffé à reflux pendant 5 h puis refroidi et filtré. Le solide obtenu est rincé avec du dichlorométhane. Le filtrat est concentré sous pression réduite. Le résidu est purifié par chromatographie sur colonne de gel de silice avec un mélange de solvants (dichlorométhane/acétate d'éthyle : 100/0 à 70/30). Les fractions de chromatographies sont partiellement concentrées. Le solide qui précipite est recueilli par filtration, lavé avec du cyclohexane et séché sous pression réduite.
18 g d'un solide blanc de 6-fluoro-1*H*-indole-2-carboxylate de méthyle sont obtenus contenant 10 à 20 % de 6-fluoro-1*H*-indole-2-carboxylate d'éthyle.

### 1.3. 6-fluoro-1-méthyl-1H-indole-2-carboxylate de méthyle

Une suspension de 7,9 g (197 mmol) d'hydrure de sodium à 60% et de 36,1 g (176 mmol) de 6-fluoro-1*H*-indole-2-carboxylate de méthyle (obtenu à l'étape 1.2.) dans 250 ml de *N,N*-diméthylformamide est agitée pendant 2 h à température ambiante. 12 ml (193 mmol) de iodométhane dans 50 ml de *N,N*-diméthylformamide sont ensuite ajoutés. Le mélange est agité à température ambiante pendant 12 h.
Le milieu réactionnel ci-dessus est versé dans un mélange d'eau et de glace. Du dichlorométhane est ajouté et la phase aqueuse est neutralisée avec de l'acide chlorhydrique (1 M). La phase organique est décantée, lavée à l'eau, séchée sur sulfate de sodium, filtrée et concentrée sous pression réduite. Le résidu est purifié par chromatographie sur colonne de gel de silice avec un mélange de solvants (cyclohexane/dichlorométhane : 50/50; puis dichlorométhane/acétate d'éthyle : 100/0 à 70/30).
37,2 g (170 mmol) d'un composé blanc de 6-fluoro-1-méthyl-1*H*-indole-2-carboxylate de méthyle sont isolés contenant 10 à 20% de 6-fluoro-1-méthyl-1 *H-*indole-2-carboxylate d'éthyle.

### 1.4. 6-fluoro-2-(méthoxycarbonyl)-1-méthyl-α-oxo-1H-indole-3-acétate d'éthyle

Une solution de 6,7 ml (60 mmol) de chlorooxoacétate d'éthyle dans 220 ml de 1,2-dichloroéthane est refroidie à 0°C. 6,6 ml (60 mmol) de tétrachlorure de titane sont ajoutés par petites portions. Le milieu réactionnel est agité pendant 30 min à 0°C. 10 g (47 mmol) de 6-fluoro-1-méthyl-1*H*-indole-2-carboxylate de méthyle (obtenu à l'étape 1.3.) sont ajoutés. L'agitation est maintenue pendant 12 h à température ambiante. Le mélange est versé dans un mélange d'eau et de glace et extrait au dichlorométhane. La phase organique est décantée, lavée à l'eau, séchée sur sulfate de sodium, filtrée et concentrée sous pression réduite. Le résidu est purifié par chromatographie sur colonne de gel de silice avec un mélange de solvants (cyclohexane/dichlorométhane : 50/50 ; puis dichlorométhane/ acétate d'éthyle : 100/0 à 90/10). Le solide est recristallisé dans un mélange de dichlorométhane et d'acétate d'éthyle.
12,1 g de solide jaunâtre de 6-fluoro-2-(méthoxycarbonyl)-1-méthyl-α-oxo-1*H-*indole-3-acétate d'éthyle sont isolés contenant 10 à 20 % de 6-fluoro-2-(éthoxycarbonyl)-1-méthyl-α-oxo-1*H*-indole-3-acétate d'éthyle.
Point de fusion : 88-91 °C

### 1.5. 7-fluoro-5-méthyl-4-oxo-3-(pyridin-2-yl)-3,5-dihydro-4H-pyridazino[4,5-b] indole-1-carboxylate d'éthyle

Une solution de 0,40 g (1,36 mmol) de 6-fluoro-2-(méthoxycarbonyl)-1-méthyl-α-oxo-*1H*-indole-3-acétate d'éthyle (obtenu à l'étape 1.4.), de 30 ml d'éthanol absolu, de quelques gouttes d'acide acétique glacial et de 0,60 g (5,5 mmol) de 2-pyridinylhydrazine est portée au reflux pendant 17 h.
Le milieu est refroidi. Un insoluble est recueilli par filtration, lavé avec de l'éther diéthylique et purifié par chromatographie sur colonne de gel de silice avec un mélange de solvants (dichlorométhane/acétate d'éthyle : 100/0 à 0/100, puis acétate d'éthyle/méthanol : 100/0 à 90/10).
Un composé (0,20 g ; 0,55 mmol) est isolé sous forme d'un solide jaune.

### 1.6. 7-fluoro-N,N,5-triméthyl-4-oxo-3-(pyridin-2-yl)-3,5-dihydro-4H-pyridazino [4,5-b]indole-1-carboxamide

Une solution de chlorhydrate de diméthylamine (0,50 g ; 6 mmol) dans 50 ml de toluène sous argon est refroidie à 0°C, puis 4 ml (8 mmol) d'une solution de triméthylaluminium (2M dans le toluène) est ajoutée par petites portions. L'agitation est maintenue 2 h à température ambiante. Le 7-fluoro-5-méthyl-4-oxo-3-(pyridin-2-yl)-3,5-dihydro-4*H*-pyridazino[4,5-*b*]indole-1-carboxylate d'éthyle sous forme solide (0,38 g ; 1,0 mmol), obtenu à l'étape 1.5., est ensuite ajouté et le milieu réactionnel est chauffé à 110°C durant 5 h.
La solution est refroidie vers 0°C et de l'eau est ajoutée goutte à goutte. Du dichlorométhane puis de l'hydroxyde de sodium à 30% sont ensuite ajoutés jusqu'à dissolution des dérivés d'aluminium. La phase organique est décantée, lavée à l'eau, séchée sur sulfate de sodium, filtrée et concentrée sous pression réduite. Le résidu est purifié par chromatographie sur colonne de gel de silice avec un mélange de solvants (dichlorométhane/acétate d'éthyle : 90/10 à 0/100, puis acétate d'éthyle/méthanol : 100/0 à 90/10). Le solide obtenu est recristallisé dans un mélange de dichlorométhane et d'acétate d'éthyle.
0,070 g (6,5 mmol) de composé sont isolés sous forme d'un solide blanc.
Point de fusion : 199 - 200°C.

### Exemple 2 (Composé N°12)

### chlorhydrate de 7-chloro-N,N,5-triméthyl-4-oxo-3-(pyridin-3-yl)-3,5-dihydro-4H-pyridazino[4,5-b]indole-1-carboxamide (1 :1)

### 2.1. 7-chloro-5-méthyl-4-oxo-3,5-dihydro-4H-pyridazino[4,5-b]indole-1-carboxylate d'éthyle

Une solution de 4,38 g (13,5 mmol) de 6-chloro-2-(éthoxycarbonyl)-1-méthyl-α-oxo-1*H*-indole-3-acétate d'éthyle (décrit dans WO-A-0044751), de 65 ml d'acide acétique glacial et de 2,7 ml (55,7 mmol) d'hydrazine monohydrate est portée au reflux pendant 3 h.
Le milieu est refroidi. Un insoluble est recueilli par filtration, lavé avec de l'eau et séché sous pression réduite.
3,58 g (11,7 mmol) de composé sont isolés sous forme d'un solide blanc.
Point de fusion : 302-303°C.

### 2.2. 7-chloro-N,N,5-triméthyl-4-oxo-3,5-dihydro-4H-pyridazino[4,5-b]indole-1-carboxamide

2,45 g (30 mmol) de chlorhydrate de diméthylamine dans 30 ml de toluène sont introduits sous argon. La solution est refroidie à 0°C, puis 15 ml (30 mmol) d'une solution de triméthylaluminium (2M dans le toluène) sont ajoutés par petites portions. Le milieu réactionnel est agité pendant 2 h à température ambiante.
30 ml (20,1 mmol) de la solution préparée précédemment sont ajoutés à une suspension de 2 g (6,5 mmol) de 7-chloro-5-méthyl-4-oxo-3,5-dihydro-4*H-*pyridazino[4,5-*b*]indole-1-carboxylate d'éthyle (obtenu à l'étape 2.1.) dans 60 ml de toluène. Le milieu réactionnel est chauffé à 100°C durant 3 h.
La solution est refroidie vers 0°C et versée dans un mélange d'une solution aqueuse d'acide chlorhydrique (1M) et de glace. Le milieu réactionnel est basifié ensuite avec une solution aqueuse d'hydroxyde de sodium (1 M). Le précipité obtenu est filtré, lavé à l'eau et séché sous pression réduite.
Un composé (1,99 g ; 6,5 mmol) est isolé sous forme d'un solide beige clair.
Point de fusion : >300°C.

### 2.3. chlorhydrate de 7-chloro-N,N,5-triméthyl-4-oxo-3-(pyridin-3-yl)-3,5-dihydro-4H-pyridazino[4,5-b]indole-1-carboxamide

0,4 g de 7-chloro-*N,N*,5-triméthyl-4-oxo-3,5-dihydro-4*H*-pyridazino[4,5-*b*]indole-1-carboxamide (1,3 mmol), obtenu à l'étape 2.2., sont dissous dans 35 ml de N-méthylpyrrolidone. 0,21 ml (2,6 mmol) de pyridine, 0,36 ml (2,6 mmol) de triéthylamine, 340 mg de tamis moléculaire, 0,47 g (2,6 mmol) d'acétate cuivrique et 0,42 g (2,6 mmol) de 2-(pyridin-3-yl)-1,3,2-dioxaborinane sont introduits à température ambiante et sous atmosphère d'argon. Après 24 h de réaction, les insolubles sont séparés par filtration et 0,21 ml (2,6 mmol) de pyridine, 0,36 ml (2,6 mmol) de triéthylamine, 340 mg de tamis moléculaire, 0,47 g (2,6 mmol) d'acétate cuivrique et 0,42 g (2,6 mmol) de 2-(pyridin-3-yl)-1,3,2-dioxaborinane sont ajoutés à la solution. L'agitation est maintenue pendant 24 h. Les insolubles sont séparés par filtration. Le solvant est éliminé sous pression réduite. Du dichlorométhane et de l'eau sont ajoutés au résidu d'évaporation. La phase aqueuse est extraite au dichlorométhane. Les phases organiques sont réunies, lavées à l'eau, séchées sur sulfate de sodium, filtrées et concentrées sous pression réduite. Le résidu est purifié par chromatographie sur colonne de silice (éluant : heptane / acétate d'éthyle: 10/90). Le solide blanc obtenu est trituré dans de l'éther diéthylique. 400 mg d'un solide blanc sont isolés.
Le chlorhydrate est formé par dissolution du solide isolé ci-dessus dans de l'éthanol et par ajout d'une solution d'acide chlorhydrique (5N) dans du propan-2-ol. Après recristallisation dans du propan-2-ol, un composé (0,35 g ; 0,84 mmol) est isolé sous forme d'un solide blanc.
Point de fusion : 228 - 230°C.

### Exemple 3 (Composé N°4)

### 7-fluoro-N,N,5-triméthyl-4-oxo-3-(2-méthoxypyridin-5-yl)-3,5-dihydro-4H-pyridazino[4,5-b]indole-1-carboxamide

### 3.1. 7-fluoro-5-méthyl-4-oxo-3,5-dihydro-4H-pyridazino[4,5-b]indole-1-carboxylate d'éthyle

Une solution de 7,80 g (26,6 mmol) de 6-fluoro-2-(méthoxycarbonyl)-1-méthyl-α-oxo-*1H*-indole-3-acétate d'éthyle (obtenu à l'étape 1.4. de l'exemple 1), de 200 ml d'acide acétique glacial et de 5 ml (103 mmol) d'hydrazine monohydrate est chauffée à 90°C pendant 20 h.
Le milieu est refroidi. Après ajout d'eau, un insoluble est recueilli par filtration, lavé avec de l'eau et séché sous pression réduite.
5,60 g (19,3 mmol) de composé sont isolés sous forme d'un solide blanc.

Point de fusion : 314-315°C.

### 3.2. 7-fluoro-N,N,5-triméthyl-4-oxo-3,5-dihydro-4H-pyridazino[4,5-b]indole-1-carboxamide

Une solution de 2,50 g (30,6 mmol) de chlorhydrate de diméthylamine dans 150 ml de toluène, sous argon, est refroidie à 0°C, puis 18 ml (36 mmol) d'une solution de triméthylaluminium (2M dans le toluène) est ajoutée par petites portions. L'agitation est maintenue pendant 2 h à température ambiante. 2,6 g (9 mmol) de 7-fluoro-5-méthyl-4-oxo-3,5-dihydro-4*H*-pyridazino[4,5-*b*]indole-1-carboxylate d'éthyle sous forme solide (obtenu à l'étape 3.1.) sont ensuite ajoutés. Le milieu réactionnel est chauffé à 110°C durant 18 h.
La solution est refroidie vers 0°C. De l'eau puis une solution 1 M d'acide chlorhydrique sont ajoutées goutte à goutte jusqu'à l'obtention d'un pH compris entre 1 et 2. Le précipité est recueilli par filtration, lavé à l'eau et séché sous pression réduite en présence de pentoxyde de phosphore.
Un composé (1,10 g ; 3,8 mmol) est isolé sous forme d'un solide blanc.
Point de fusion : >300°C.

### 3.3. 7-fluoro-N,N,5-triméthyl-4-oxo-3-(2-méthoxypyridin-5-yl)-3,5-dihydro-4H-pyridazino[4,5-b]indole-1-carboxamide

Une solution de 0,3 g (1,04 mmol) de 7-fluoro-*N*,*N*,5-triméthyl-4-oxo-3,5-dihydro-4*H*-pyridazino[4,5-*b*]indole-1-carboxamide (obtenu à l'étape 3.2.), de 0,120 g (0,63 mmol) d'iodure cuivreux, de 0,20 g (1,45 mmol) de carbonate de potassium et de 0,60 g (3,19 mmol) de 3-bromo-6-méthoxypyridine dans 50 ml de *N,N-*diméthylformamide est chauffée à 150°C pendant 20 h. Le mélange réactionnel est refroidi et concentré sous pression réduite. Du dichlorométhane, de l'eau et une solution d'hydroxyde de sodium (1M) sont ajoutés. La phase organique est décantée, lavée à l'eau, séchée sur sulfate de sodium, filtrée et concentrée sous pression réduite. Le résidu est purifié par chromatographie sur colonne de gel de silice (éluant : dichlorométhane/acétate d'éthyle: 100/0 à 0/100; puis acétate d'éthyle/méthanol : 95/5). Le solide blanc obtenu est recristallisé dans un mélange dichlorométhane / acétate d'éthyle et lavé avec de l'éther diéthylique. Un solide blanc (0,26 g ; 0,66 mmol) est isolé.
Point de fusion : 225 - 226°C.

### Exemple 4 (Composé N°9)

### 7-fluoro-N,5-diméthyl-4-oxo-3-(pyridin-3-yl)-3,5-dihydro-4H-pyridazino[4,5-b]indole-1-carboxamide

### 4.1. 7-fluoro-5-méthyl-4-oxo-3-(pyridin-3-yl)-3,5-dihydro-4H-pyridazino[4,5-b]indole-1-carboxylate d'éthyle

Le 7-fluoro-5-méthyl-4-oxo-3,5-dihydro-4*H*-pyridazino[4,5-*b*]indole-1-carboxylate d'éthyle (0,9 g ; 3,11 mmol), obtenu à l'étape 3.1. de l'exemple 3, est dissous dans 60 ml de *N*-méthylpyrrolidone. 0,50 ml (6,2 mmol) de pyridine, 0,8 ml (5,7 mmol) de triéthylamine, 4 g de tamis moléculaire, 1,0 g (5,5 mmol) d'acétate cuivrique et 0,90 g (5,5 mmol) de 2-(3-pyridinyl)-1,3,2-dioxaborinane sont introduits à température ambiante et sous atmosphère d'argon. Après 24 h de réaction, le solvant est éliminé sous pression réduite. Du dichlorométhane, de l'eau et de la soude (1 M) sont ajoutés. Les insolubles sont séparés par filtration, la phase organique est décantée et la phase aqueuse est extraite au dichlorométhane. Les phases organiques sont réunies, lavées à l'eau, séchées sur sulfate de sodium, filtrées et concentrées sous pression réduite. Le résidu est purifié par chromatographie sur colonne de gel de silice (éluant : dichlorométhane/acétate d'éthyle : 100/0 à 0/100 ; acétate d'éthyle/méthanol : 100/0 à 90/10).
Un solide blanc (0,57 g) est isolé.
Point de fusion : 214 - 215°C.

### 4.2. 7-fluoro-N,5-diméthyl-4-oxo-3-(pyridin-3-yl)-3,5-dihydro-4H-pyridazino[4,5-b]indole-1-carboxamide

Un courant de méthylamine gazeuse est introduit dans une solution de 0,28 g (0,76 mmol) de 7-fluoro-5-méthyl-4-oxo-3-(pyridin-3-yl)-3,5-dihydro-4*H-*pyridazino[4,5-*b*]indole-1-carboxylate d'éthyle (obtenu à l'étape 4.1.) dans 30 ml de dichlorométhane et 70 ml de méthanol. L'agitation est maintenue pendant 4 h. Le milieu réactionnel est concentré sous pression réduite. Le résidu est purifié par chromatographie sur colonne de gel de silice (éluant : dichlorométhane/méthanol : 100/0 à 90/10). Le solide obtenu est recristallisé dans un mélange de dichlorométhane et d'acétate d'éthyle.
Un solide blanc (0,22 g) est isolé.
Point de fusion : 270 - 272°C.

### Exemple 5 (Composé N°6)

### 1-[[7-fluoro-5-méthyl-3-(pyrimidin-2-yl)-4-oxo-3,5-dihydro-4H-pyridazino[4,5-b]indol-1-yl]carbonyl]pyrrolidine

### 5.1. 1-[[7-fluoro-5-méthyl-4-oxo-3,5-dihydro-4H-pyridazino[4,5-b]indol-1-yl] carbonyl]pyrrolidine

12 ml (24 mmol) d'une solution de triméthylaluminium (2M dans le toluène) dans 100 ml de toluène, sous argon, est refroidie à 0°C, puis 2 ml (24 mmol) de pyrrolidine sont ajoutés par petites portions. L'agitation est maintenue pendant 2 h à température ambiante. 2,0 g (6,9 mmol) de 7-fluoro-5-méthyl-4-oxo-3,5-dihydro-4*H*-pyridazino[4,5-*b*]indole-1-carboxylate d'éthyle (obtenu à l'étape 3.1. de l'exemple 3) sous forme solide sont ensuite ajoutés. Le milieu réactionnel est chauffé à 110°C durant 18 h.
La solution est refroidie vers 0°C. De l'eau, puis une solution d'acide chlorhydrique (1M ) sont ajoutés goutte à goutte jusqu'à l'obtention d'un pH compris entre 1 et 2.
Le précipité obtenu est filtré, lavé à l'eau et séché sous pression réduite en présence de pentoxyde de phosphore.
Un composé (1,50 g ; 4,6 mmol) est isolé sous forme d'un solide blanc.
Point de fusion : >300°C.

### 5.2. 1-[[7-fluoro-5-méthyl-3-(pyrimidin-2-yl)-4-oxo-3,5-dihydro-4H-pyridazino [4,5-b]indol-1-yl]carbonyl]pyrrolidine

Une solution de 0,24 g (0,73 mmol) de 1-[[7-fluoro-5-méthyl-4-oxo-3,5-dihydro-4*H-*pyridazino[4,5-*b*]indol-1-yl]carbonyl]pyrrolidine (obtenu à l'étape 5.1.), de 0,120 g (0,63 mmol) d'iodure cuivreux, de 0,15 g (1,09 mmol) de carbonate de potassium et de 0,30 g (1,89 mmol) de 2-bromopyrimidine dans 40 ml de *N*,*N-*diméthylformamide est chauffée à 150°C pendant 16 h. Le mélange réactionnel est refroidi et concentré sous pression réduite. Du dichlorométhane, de l'eau et une solution concentrée d'hydroxyde de sodium sont ajoutés. La phase organique est décantée, filtrée sur Célite®, lavée à l'eau, séchée sur sulfate de sodium, filtrée et concentrée sous pression réduite. Le résidu est purifié par chromatographie sur colonne de gel de silice (éluant : dichlorométhane/acétate d'éthyle: 80/20 à 0/100; acétate d'éthyle/méthanol : 100/0 à 90/10). Le solide obtenu est recristallisé dans un mélange dichlorométhane/acétate d'éthyle et lavé avec de l'éther diéthylique.
Un solide blanc (0,04 g ; 0,10 mmol) est isolé.
Point de fusion : 238 - 239°C.

### Exemple 6 (Composé N°26)

### chlorhydrate de 4-méthyl-1-[[7-chloro-5-méthyl-3-(pyridin-3-yl)-4-oxo-3,5-dihydro-4H-pyridazino[4,5-b]indol-1-yl]carbonyl]pipérazine (1:1)

### 6.1. 4-méthyl-1-[[7-chloro-5-méthyl-4-oxo-3,5-dihydro-4H-pyridazino[4,5-b]indol-1-yl]carbonyl]pipérazine

Une solution de 4-méthylpipérazine (1,1 ml ; 10 mmol) dans 10 ml de toluène est refroidie à 0°C sous argon. 5 ml (10 mmol) d'une solution de triméthylaluminium (2M dans le toluène) sont ajoutés par petites portions. L'agitation est maintenue pendant 2 h à température ambiante. 1,0 g (3,27 mmol) de 7-chloro-5-méthyl-4-oxo-3,5-dihydro-4*H*-pyridazino[4,5-*b*]indole-1-carboxylate d'éthyle (obtenu à l'étape 2.1. de l'exemple 2) dans 30 ml de toluène est ensuite ajouté. Le milieu réactionnel est chauffé à 110°C durant 2 h.
La solution est refroidie vers 0°C. De l'eau, puis une solution de soude concentrée sont ajoutées goutte à goutte. Le précipité est filtré, lavé à l'eau et séché sous pression réduite en présence de pentoxyde de phosphore.
Un composé (0,89 g) est isolé sous forme d'un solide blanc.

### 6.2. chlorhydrate de 4-méthyl-1-[[7-chloro-5-méthyl-3-(pyridin-3-yl)-4-oxo-3,5-dihydro-4H-pyridazino[4,5-b]indol-1-yl]carbonyl]pipérazine (1:1)

La 4-méthyl-1-[[7-chloro-5-méthyl-4-oxo-3,5-dihydro-4*H*-pyridazino[4,5-*b*]indol-1-yl]carbonyl]pipérazine (0,75 g ; 2,1 mmol), obtenue à l'étape 6.1., est dissoute dans 60 ml de *N*-méthylpyrrolidone. 0,39 ml (4,8 mmol) de pyridine, 0,67 ml (4,8 mmol) de triéthylamine, 800 mg de tamis moléculaire, 0,87 g (4,8 mmol) d'acétate cuivrique et 0,78 g (4,8 mmol) de 2-(pyridin-3-yl)-1,3,2-dioxaborinane sont introduits à température ambiante et sous atmosphère d'argon. Après 24 h de réaction, les insolubles sont séparés par filtration. 0,39 ml (4,8 mmol) de pyridine, 0,67 ml (4,8 mmol) de triéthylamine, 2,0 g de tamis moléculaire, 0,87 g (4,8 mmol) d'acétate cuivrique et 0,78 g (4,8 mmol) de 2-(pyridin-3-yl)-1,3,2-dioxaborinane sont ajoutés à la solution. L'agitation est prolongée pendant encore 24h. Le solvant est éliminé sous pression réduite. Du dichlorométhane et de l'eau sont ajoutés. Les insolubles sont séparés par filtration. La phase organique est décantée et la phase aqueuse est extraite au dichlorométhane. Les phases organiques sont réunies, lavées à l'eau, séchées sur sulfate de sodium, filtrées et concentrées sous pression réduite. Le résidu est trituré dans de l'éther diisopropylique. Le précipité obtenu est collecté par filtration et purifié par chromatographie sur colonne de gel de silice (éluant : dichlorométhane / méthanol : 100/0 à 90/10). Un solide couleur crème (0,3 g) est isolé.
Le chlorhydrate est formé par dissolution du solide dans du propan-2-ol et par ajout de 7 ml d'une solution d'acide chlorhydrique (0,1 N) dans du propan-2-ol.
Après recristallisation dans du propan-2-ol, un composé (0,23 g ; 0,44 mmol) est isolé sous forme d'un solide blanc.
Point de fusion : 267 - 268°C (décomposition).

### Exemple 7 (Composé N°11)

### 7-chloro-N,N,5-triméthyl-4-oxo-3-(pyridin-2-yl)-3,5-dihydro-4H-pyridazino[4,5-b]indole-1-carboxamide

0,4 g de 7-chloro-*N*,*N*,5-triméthyl-4-oxo-3,5-dihydro-4*H*-pyridazino[4,5-*b*]indole-1-carboxamide (1,3 mmol), obtenu à l'étape 2.2. de l'exemple 2, sont dissous dans 35 ml de *N*-méthylpyrrolidone. 0,2 ml (2,6 mmol) de pyridine, 0,36 ml (2,6 mmol) de triéthylamine, 420 mg de tamis moléculaire, 0,48 g (2,6 mmol) d'acétate cuivrique et 0,874 g (2,6 mmol) d'un mélange [1:1] de tripropoxypyridin-2-ylboronate de lithium et de propanol sont introduits à température ambiante et sous atmosphère d'argon. Après 24 h de réaction, 0,2 ml (2,6 mmol) de pyridine, 0,36 ml (2,6 mmol) de triéthylamine, 420 mg de tamis moléculaire, 0,48 g (2,6 mmol) d'acétate cuivrique et 0,874 g (2,6 mmol) d'un mélange [1:1] de tripropoxypyridin-2-ylboronate de lithium et de propanol sont ajoutés à la solution. L'agitation est prolongée pendant 24 h supplémentaires. Le solvant est évaporé. Du dichlorométhane et de l'eau sont ajoutés. La phase organique est décantée et la phase aqueuse est extraite au dichlorométhane. Les phases organiques sont réunies, séchées sur sulfate de sodium, filtrées et concentrées sous pression réduite. Le résidu est purifié par chromatographie sur colonne de gel de silice (éluant : dichlorométhane/méthanol : 100/0 à 95/5). Le solide obtenu est recristallisé dans l'éthanol.
Un solide beige clair (0,95 g) est isolé.
Point de fusion : 210 - 211 °C.

### Exemple 8 (Composé N°14)

### chlorhydrate de 7-chloro-N,N,5-triméthyl-4-oxo-3-(pyridin-4-yl)-3,5-dihydro-4H-pyridazino[4,5-b]indole-1-carboxamide (1 :1)

0,4 g de 7-chloro-*N*,*N*,5-triméthyl-4-oxo-3,5-dihydro-4*H*-pyridazino[4,5-*b*]indole-1-carboxamide (1,3 mmol), obtenu à l'étape 2.2. de l'exemple 2, sont dissous dans 35 ml de *N*-méthylpyrrolidone. 0,2 ml (2,6 mmol) de pyridine, 0,4 ml (2,7 mmol) de triéthylamine, 300 mg de tamis moléculaire, 0,48 g (2,6 mmol) d'acétate cuivrique et 0,32 g (2,6 mmol) d'acide pyridin-4-ylboronique sont introduits à température ambiante et sous atmosphère d'argon. Après 24 h de réaction, les insolubles sont séparés par filtration et 0,2 ml (2,6 mmol) de pyridine, 0,4 ml (2,7 mmol) de triéthylamine, 300 mg de tamis moléculaire, 0,48 g (2,6 mmol) d'acétate cuivrique et 0,32 g (2,6 mmol) d'acide pyridin-4-ylboronique sont ajoutés à la solution. L'agitation est maintenue pendant 24 h. Les insolubles sont séparés par filtration. Le solvant est éliminé sous pression réduite. Du dichlorométhane et de l'eau sont ajoutés au résidu d'évaporation. La phase aqueuse est extraite au dichlorométhane. Les phases organiques sont réunies, lavées à l'eau, séchées sur sulfate de sodium, filtrées et concentrées sous pression réduite. Le résidu est purifié par chromatographie sur colonne de gel de silice (éluant : cyclohexane /acétate d'éthyle: 10/90, puis dichlorométhane / méthanol : 95/5). Le solide blanc obtenu est trituré dans de l'éther diéthylique. 350 mg de 7-chloro-*N,N*,5-triméthyl-4-oxo-3-(pyridin-4-yl)-3,5-dihydro-4*H*-pyridazino[4,5-*b*]indole-1-carboxamide (composé N°13) sont isolés sous forme d'un solide blanc.
Point de fusion : 276 - 278°C
Le chlorhydrate est formé par dissolution du solide isolé ci-dessus dans de l'éthanol et par ajout d'une solution d'acide chlorhydrique (5N) dans du propan-2-ol. Après recristallisation dans du propan-2-ol, un composé (0,30 g ; 0,72 mmol) est isolé sous forme d'un solide blanc.
Point de fusion : 263 - 265°C.

### Exemple 9 (Composé N°37)

### 7-chloro-N,N,5-triméthyl-3-(1-oxydopyridin-4-yl)-4-oxo-3,5-dihydro-4H-pyridazino[4,5-b]indole-1-carboxamide

0,35 g (0,92 mmol) de 7-chloro-*N,N*,5-triméthyl-4-oxo-3-(pyridin-4-yl)-3,5-dihydro-4*H*-pyridazino[4,5-*b*]indole-1-carboxamide, obtenu à l'exemple 8, sont dissous dans 30 ml d'acide acétique glacial. 3,5 g (36 mmol) d'une solution de peroxyde d'hydrogène (à 35% dans l'eau) sont lentement ajoutés. Le mélange réactionnel est chauffé à 80°C pendant 30 h puis refroidi à température ambiante. De l'eau puis de l'hydrogénocarbonate de sodium sont ajoutés jusqu'à obtenir un pH d'environ 8. La phase aqueuse est extraite au dichlorométhane. La phase organique est lavée avec de l'eau, séchée sur sulfate de sodium, filtrée et concentrée sous pression réduite. Le résidu est purifié par chromatographie sur colonne de gel de silice (éluant : dichlorométhane/méthanol : 98/2 à 80/20). Le solide obtenu est recristallisé dans du méthanol. On obtient 100 mg de composé sous forme d'un solide blanc.
Point de fusion : 301 - 304°C

### Exemple 10 (Composé N°38)

### 7-chloro-3-(2-méthoxypyridin-4-yl)-N,N,5-triméthyl-4-oxo-3,5-dihydro-4H-pyridazino[4,5-b]indole-1-carboxamide

### 10.1. 3-(2-bromopyridin-4-yl)-7-chloro-N,N,5-triméthyl-4-oxo-3,5-dihydro-4H-pyridazino[4,5-b]indole-1-carboxamide

0,34 g (0,85 mmol) de 7-chloro-*N,N*,5-triméthyl-3-(1-oxydopyridin-4-yl)-4-oxo-3,5-dihydro-4*H*-pyridazino[4,5-*b*]indole-1-carboxamide, obtenu à l'exemple 9, sont dissous dans 50 ml de dichlorométhane, sous atmosphère inerte. 0,24 ml (1,7 mmol) de triéthylamine sont ajoutés. Le mélange est refroidi par un bain d'eau glacée et 0,49 g (1,7 mmol) d'oxybromure de phosphore sont ajoutés par petites portions. Le milieu réactionnel est agité pendant 30 minutes à température ambiante puis chauffé pendant 2 h 30 à reflux et enfin refroidi à température ambiante. Il est ensuite versé sur de la glace pilée. Du dichlorométhane et une solution aqueuse (1M) d'hydroxyde de sodium sont ajoutés jusqu'à un pH basique. La phase organique est décantée, lavée avec de l'eau, séchée sur sulfate de sodium, filtrée et concentrée sous pression réduite. Le résidu est purifié par chromatographie sur colonne de gel de silice (éluant : dichlorométhane/ acétate d'éthyle : 80/20 à 0/100). On isole 180 mg d'un solide blanc. Ce composé est utilisé tel quel pour l'étape suivante.

### 10.2. 7-chloro-3-(2-méthoxypyridin-4-yl)-N,N,5-triméthyl-4-oxo-3,5-dihydro-4H-pyridazino[4,5-b]indole-1-carboxamide

120 mg (5,2 mmol) de sodium sont ajoutés sous atmosphère inerte dans 20 ml de méthanol. 180 mg (0,39 mmol) de 3-(2-bromopyridin-4-yl)-7-chloro-*N*,*N*,5-triméthyl-4-oxo-3,5-dihydro-4*H*-pyridazino[4,5-*b*]indole-1-carboxamide, obtenu à l'exemple 10.1, et 20 ml de *N,N*-diméthylformamide sont ajoutés. La solution est chauffée à 80°C pendant 14 h. Elle est refroidie à température amiante, puis concentrée sous pression réduite. De l'eau et du dichlorométhane sont ajoutés au résidu. La phase organique est décantée, lavée avec de l'eau, séchée sur sulfate de sodium, filtrée et concentrée sous pression réduite. Le résidu est purifié par chromatographie sur colonne de gel de silice (éluant : dichlorométhane/acétate d'éthyle : 80/20 à 0/100). Le solide obtenu est recristallisé dans un mélange dichlorométhane / acétate d'éthyle puis rincé à l'éther diéthylique. 60 mg d'un solide blanc sont isolés.
Point de fusion : 237 - 238°C

Le tableau qui suit illustre les structures chimiques et les propriétés physiques de quelques composés de l'invention.
Dans la colonne "Sel" de ce tableau, "HCl" désigne un chlorhydrate, "-" désigne un composé à l'état de base. Les rapports molaires acide : base sont indiqués en regard.

**Tableau**

| | | | | | | |
|---|---|---|---|---|---|---|
| 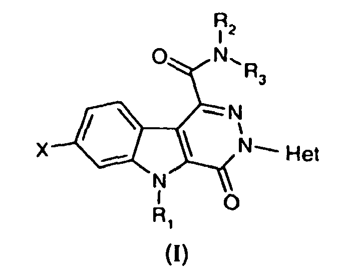 | | | | | | |

| **N° composé** | **X** | **R**_{**1**} | **NR**_{**2**}**R**_{**3**} | **Het** | **Sel** | **P.F. (C°)** |
|---|---|---|---|---|---|---|
| 1 | F | CH₃ | N(CH₃)₂ | pyridin-2-yl | - | 199 - 200 |
| 2 | F | CH₃ | N(CH₃)₂ | pyridin-3-yl | - | 220 - 221 |
| 3 | F | CH₃ | N(CH₃)₂ | pyridin-4-yl | HCl 1:1 | 266 - 271 |
| 4 | F | CH₃ | N(CH₃)₂ | 2-méthoxypyridin-5-yl | - | 225 - 226 |
| 5 | F | CH₃ | N(CH₃)₂ | quinolin-3-yl | - | 237 - 238 |
| 6 | F | CH₃ | 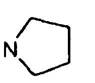 | pyrimidin-2-yl | - | 238 - 239 |
| 7 | F | CH₃ | 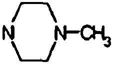 | pyridin-3-yl | HCl 1:1 | 269 - 273 |
| 8 | F | CH₃ | 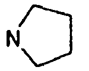 | pyridin-3-yl | - | 220 - 221 |
| 9 | F | CH₃ | NH(CH₃) | pyridin-3-yl | - | 270 - 272 |
| 10 | F | CH₃ | NH₂ | pyridin-3-yl | - | 319 - 320 |
| 11 | Cl | CH₃ | N(CH₃)₂ | pyridin-2-yl | - | 210 - 211 |
| 12 | Cl | CH₃ | N(CH₃)₂ | pyridin-3-yl | HCl 1:1 | 228-230 |
| 13 | Cl | CH₃ | N(CH₃)₂ | pyridin-4-yl | - | 276 - 278 |
| 14 | Cl | CH₃ | N(CH₃)₂ | pyridin-4-yl | HCl 1:1 | 263 - 265 |
| 15 | Cl | CH₃ | N(CH₃)₂ | 5-méthylpyridin-2-yl | - | 222 - 224 |
| 16 | Cl | CH₃ | N(CH₃)₂ | 2-méthoxypyridin-5-yl | - | 236 - 237 |
| 17 | Cl | CH₃ | N(CH₃)₂ | 2-méthylpyridin-5-yl | - | 214 - 216 |
| 18 | Cl | CH₃ | N(CH₃)₂ | 2-bromopyridin-5-yl | - | 250 - 252 |
| 19 | Cl | CH₃ | N(CH₃)₂ | quinolin-3-yl | - | 271 - 272 |
| 20 | Cl | CH₃ | N(CH₃)₂ | isoquinolin-4-yl | - | 189 - 191 |
| 21 | Cl | CH₃ | N(CH₃)₂ | 6-méthylpyridazin-3-yl | - | 238 - 239 |
| 22 | Cl | CH₃ | N(CH₃)₂ | pyrimidin-5-yl | - | 251 - 252 |
| 23 | Cl | CH₃ | N(CH₃)₂ | pyrimidin-2-yl | - | 272 - 274 |
| 24 | Cl | CH₃ | N(CH₃)₂ | pyrazin-2-yl | - | 231 - 232 |
| 25 | Cl | CH₃ | 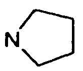 | pyridin-3-yl | HCl 1:1 | 232 - 234 |
| 26 | Cl | CH₃ | 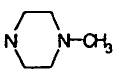 | pyridin-3-yl | HCl 1:1 | 267 - 268 |
| 27 | Cl | CH₃ | 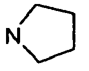 | pyridin-4-yl | - | 236 - 237 |
| 28 | Cl | CH₃ | NH(CH₃) | pyridin-4-yl | - | 321 - 323 |
| 29 | Cl | CH₃ | NH₂ | pyridin-4-yl | - | 341 - 346 |
| 30 | Cl | CH₃ | N(CH₃)₂ | 4-méthoxypyridin-2-yl | - | 243 - 244 |
| 31 | Cl | CH₃ | 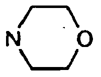 | pyridin-4-yl | | 307 - 308 |
| 32 | Cl | CH₃ | N(CH₂CH₃)₂ | pyridin-4-yl | - | 217 - 218 |
| 33 | Cl | CH₃ | NCH₃(CH₂CH₃) | pyridin-4-yl | - | 211 - 213 |
| 34 | Cl | CH₃ | 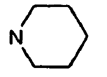 | pyridin-4-yl | - | 242 - 243 |
| 35 | Cl | CH₃ | N(CH₃)₂ | 2-méthylpyridin-4-yl | - | 267 - 269 |
| 36 | Cl | CH₃ | 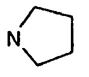 | 2-méthylpyridin-4-yl | - | 246 - 247 |
| 37 | Cl | CH₃ | N(CH₃)₂ | 1-oxydopyridin-4-yl | - | 301 - 304 |
| 38 | Cl | CH₃ | N(CH₃)₂ | 2-méthoxypyridin-4-yl | - | 237 - 238 |

Les composés de l'invention ont été soumis à des essais pharmacologiques qui ont mis en évidence leur intérêt comme substances à activités thérapeutiques.

Les composés de l'invention présentent également des caractéristiques de solubilité dans l'eau qui favorise une bonne activité in vivo.

### Etude de la liaison [³H]Ro5-4864 aux récepteurs de type périphérique aux benzodiazépines (sites p ou PBR)

L'affinité des composés de l'invention pour les sites p ou PBR (sites de liaison de type périphérique aux benzodiazépines) a été déterminée.
Les récepteurs sites p peuvent être marqués sélectivement dans des membranes de rein de rat incubées en présence de [³H]Ro5-4864. Les composés de l'invention ont fait l'objet d'une étude *in vitro* quant à leur affinité pour ces récepteurs.
Les animaux utilisés sont des rats mâles Sprague Dawley (Iffa Credo) de 180 à 300 mg. Après décapitation, le rein est prélevé et le tissu est homogénéisé à 4°C au moyen d'un homogénéiseur Polytron^{™} pendant 2 min à 6/10 de la vitesse maximale, dans 35 volumes de tampon phosphate Na₂HPO₄ 50 mM, à un pH ajusté à 7,5 avec du NaH₂PO₄. L'homogénat membranaire est filtré sur gaze et dilué 10 fois avec du tampon.
Le [³H]Ro5-4864 (Activité spécifique : 70-90 Ci/mmol ; New England Nuclear), à une concentration de 0,5 nM, est incubé en présence de 100 µl de l'homogénat membranaire dans un volume final de 1 ml de tampon contenant le composé à tester.
Après une incubation de 3 h à 0°C, les membranes sont récupérées par filtration sur filtres Whatman GF/B^{™} lavés avec 2 fois 4,5 ml de tampon d'incubation froid (0°C). La quantité de radioactivité retenue par le filtre est mesurée par scintigraphie liquide.
Pour chaque concentration de composé étudié, le pourcentage d'inhibition de la liaison du [³H]Ro5-4864, puis la concentration CI₅₀, concentration qui inhibe 50% de la liaison spécifique, sont déterminés.
Les CI₅₀ des composés les plus actifs de l'invention présentent des valeurs allant de 1 nM à 200 nM.

Les composés de l'invention sont donc des ligands affins pour les récepteurs de type périphérique aux benzodiazépines.

### Etude de l'activité neurotrophe

### Test de survie des motoneurones après section du nerf facial chez le rat âgé de 4 jours

Après lésion du nerf facial chez le rat immature, les motoneurones du noyau facial subissent une mort neuronale par apoptose. L'évaluation de la survie neuronale est réalisée à l'aide de méthodes histologiques et comptage neuronal.
Des rats immatures de 4 jours sont anesthésiés au pentobarbital (3 mg/kg par voie i.p.). Le nerf facial droit est dégagé et sectionné, à sa sortie du foramen stylomastoïdien. Après le réveil, les ratons sont remis avec leur mère et traités, pendant 7 jours, par une ou deux administrations quotidiennes, par voie orale ou intrapéritonéale, à des doses allant de 1 à 10 mg/kg.
7 jours après la lésion, les animaux sont décapités, et les cerveaux congelés dans l'isopentane à -40°C. Le noyau facial est coupé au cryostat, en sections de 10 µm, dans sa totalité. Les motoneurones sont colorés au crésyl violet et comptés à l'aide du logiciel Histo^{™} (Biocom^{™}).
Dans ce modèle, les composés de l'invention augmentent la survie neuronale d'environ 10 à 30%.

Les résultats des essais montrent que les composés les plus actifs de l'invention favorisent la régénération nerveuse.

Les composés de l'invention peuvent donc entrer dans la composition d'un médicament.

Ils peuvent être utilisés pour la préparation de médicaments destinés à la prévention et/ou au traitement des neuropathies périphériques de différent types, comme les neuropathies traumatiques ou ischémiques, neuropathies infectieuses, alcooliques, médicamenteuses ou génétiques, ainsi que des affections du motoneurone, telle que les amyotrophies spinales et la sclérose latérale amyotrophique. Ces médicaments trouveront également une application dans le traitement des maladies neurodégénératives du système nerveux central, soit de type aigu comme les accidents vasculaires cérébraux et les traumatismes crâniens et médullaires, soit de type chronique comme les maladies auto-immunes (sclérose en plaques), la maladie d'Alzheimer, la maladie de Parkinson et toute autre maladie dans laquelle l'administration de facteurs neurotrophes est censée avoir un effet thérapeutique.

Les composés de l'invention peuvent également être utilisés pour la préparation de médicaments destinés à la prévention et/ou au traitement de l'anxiété, de l'épilepsie et des troubles du sommeil. En effet, les ligands des sites p ou PBR stimulent la production de neurostéroides tels que la pregnénolone, la déhydroepiandrostérone, et la 3-alphahydroxy-5-alphaprégnan-20-one en favorisant le transfert du cholestérol de l'extérieur à l'intérieur de la membrane mitochondriale. Ces neurostéroides modulent l'activité du complexe macromoléculaire GABA_{A}-canal chlorure et ainsi peuvent produire des activités anxiolytiques, anti-convulsivantes et sédatives (D. Bitran et al., *Psychopharmacology* **2000,** 151, 64-71 ; S. Okuyama et al., *Life Sci*., **1999,** 64 (16), 1455-1464 ; L.D. McCauley et al., *Eur. J. Pharmacol*., **1995,** 276, 145-153 ; S.K. Kulkarni et al., *Drugs of today*, **1995,** 31, 433-4558).

Les composés de l'invention peuvent aussi être utilisés dans les traitements de l'insuffisance rénale aiguë ou chronique, de la glomérulonéphrite, de la néphropathie diabétique, de l'ischémie et de l'insuffisance cardiaques, de l'infarctus du myocarde, de l'ischémie des membres inférieurs, du vasospasme coronaire, de l'angine de poitrine, des pathologies associées aux valves cardiaques, des maladies cardiaques inflammatoires, des effets secondaires dus à des médicaments cardiotoxiques ou aux suites d'une chirurgie cardiaque, de l'athérosclérose et de ses complications thrombo-emboliques, de la resténose, des rejets de greffes, des conditions liées à une prolifération ou une migration incorrectes des cellules musculaires lisses.

Par ailleurs, des données récentes de la littérature indiquent que le récepteur de type périphérique aux benzodiazépines pourrait jouer un rôle fondamental dans la régulation de la prolifération cellulaire et les processus de cancérisation. D'une manière générale, et par comparaison avec des tissus normaux, une densité accrue de récepteurs de type périphérique aux benzodiazépines est observée dans différents types de tumeurs et cancers.

Dans les astocytomes humains, le niveau d'expression du récepteur de type périphérique aux benzodiazépines est corrélé avec le degré de malignité de la tumeur, l'index de prolifération et la survie des patients. Dans les tumeurs cérébrales humaines, l'augmentation du nombre de récepteurs de type périphérique aux benzodiazépines est utilisée comme une indication diagnostique en imagerie médicale et comme cible thérapeutique pour des conjugués formés d'un ligand du récepteur de type périphériques aux benzodiazépines et d'une drogue cytostatique. Une densité élevée de récepteurs de type périphérique aux benzodiazépines est également observée dans les carcinomes ovariens et les cancers du sein. Concernant ces derniers, il a été démontré que le niveau d'expression des récepteurs de type périphérique aux benzodiazépines est relié au potentiel agressif de la tumeur ; de plus la présence d'un agoniste du récepteur de type périphérique aux benzodiazépines stimule la croissance d'une lignée de cancer mammaire.

L'ensemble de ces résultats, qui suggère une fonction délétère du récepteur de type périphérique aux benzodiazépines dans les processus de cancérisation, constitue une base pertinente pour la recherche de ligands synthétiques spécifiques du récepteur de type périphérique aux benzodiazépines capables d'en bloquer les effets.

Les composés peuvent donc être utilisés pour le traitement des tumeurs et cancers.

Les récepteurs de type périphérique aux benzodiazépines sont également présents au niveau de la peau et, à ce titre, les composés utilisables selon l'invention peuvent être utilisés pour la prophylaxie ou le traitement des stress cutanés.
Par stress cutané, on entend les différentes situations qui pourraient provoquer des dommages en particulier au niveau de l'épiderme, quel que soit l'agent qui provoque ce stress. Cet agent peut être interne et/ou externe à l'organisme, comme un agent chimique ou radicalaire, ou bien externe, comme un rayonnement ultraviolet.
Ainsi les composés utilisables selon l'invention sont destinés à prévenir et à lutter contre les irritations cutanées, les dartres, les érythèmes, les sensations dysesthésiques, les sensations d'échauffement, les prurits de la peau et/ou des muqueuses, le vieillissement et peuvent aussi être utilisés dans les désordres cutanés tels que, par exemple, le psoriasis, les maladies prurigineuses, l'herpès, les photodermatoses, les dermatites atopiques, les dermatites de contact, les lichens, les prurigos, les prurits, les piqûres d'insectes, dans les fibroses et autres troubles de la maturation des collagènes, dans les désordres immunologiques ou encore dans des affections dermatologiques comme l'eczéma.

Les composés de l'invention peuvent également être utilisés pour la prévention et le traitement des maladies inflammatoires chroniques, notamment la polyarthrite rhumatoïde, et des maladies inflammatoires pulmonaires, en particulier l'asthme, le syndrome de détresse respiratoire aiguë (ARDS, acute respiratory distress syndrome) et la maladie obstructive du poumon (COPD, chronic obstructive pulmonary disease), la mucovicidose, les maladies bronchopulmonaires, les pneumopathies, la fibrose pulmonaire.

Ainsi l'invention a pour objet des compositions pharmaceutiques contenant une dose efficace d'au moins un composé de formule générale (I), à l'état de base, de sel, de solvat ou d'hydrate pharmaceutiquement acceptables, et en mélange, le cas échéant, avec des excipients convenables.
Lesdits excipients sont choisis selon la forme pharmaceutique et le mode d'administration souhaité.
Les compositions pharmaceutiques de l'invention peuvent ainsi être destinées à l'administration orale, sublinguale, sous-cutanée, intramusculaire, intraveineuse, topique, intratrachéale, intranasale, transdermique, rectale, intraoculaire.
Les formes unitaires d'administration peuvent être, par exemple, des comprimés, des gélules, des granules, des poudres, des solutions ou suspensions orales ou injectables, des timbres transdermiques ("patch"), des suppositoires. Pour l'administration topique on peut envisager des pommades, lotions et collyres.

Lesdites formes unitaires sont dosées pour permettre une administration journalière de 0,001 à 20 mg de principe actif par kg de poids corporel, selon la forme galénique.

Pour préparer des comprimés on ajoute au principe actif, micronisé ou non, un véhicule pharmaceutique qui peut être composé de diluants, comme par exemple le lactose, la cellulose microcristalline, l'amidon, et des adjuvants de formulation comme des liants, (polyvinylpyrrolidone, hydroxypropylméthylcellulose, etc), des agents d'écoulement comme la silice, des lubrifiants comme le stéarate de magnésium, l'acide stéarique, le tribehenate de glycerol, le stéarylfumarate de sodium. Des agents mouillants ou tensioactifs tels que le laurylsulfate de sodium peuvent aussi être ajoutés.
Les techniques de réalisation peuvent être la compression directe, la granulation sèche, la granulation humide ou la fusion à chaud.
Les comprimés peuvent être nus, dragéifiés, par exemple par du saccharose, ou enrobés avec divers polymères ou autres matières appropriées. II peuvent être conçus pour permettre une libération rapide, retardée ou prolongée du principe actif grâce à des matrices polymères ou à des polymères spécifiques utilisés dans l'enrobage.

Pour préparer des gélules on mélange le principe actif avec des véhicules pharmaceutiques secs (simple mélange, granulation sèche ou humide, ou fusion à chaud), liquides ou semi-solides.
Les gélules peuvent être dures ou molles, pelliculées ou non, de manière à avoir une activité rapide, prolongée ou retardée (par exemple pour une forme entérique).

Une composition sous forme de sirop ou d'élixir ou pour l'administration sous forme de gouttes peut contenir le principe actif conjointement à un édulcorant, de préférence acalorique, du méthylparaben ou du propylparaben comme antiseptique, un agent de sapidité et un colorant.

Les poudres et granules dispersibles dans de l'eau peuvent contenir le principe actif en mélange avec des agents de dispersion ou des agents mouillants, ou des agents dispersants comme la polyvinylpyrrolidone, de même qu'avec des édulcorants et des agents correcteurs de goût.

Pour l'administration rectale, on recourt à des suppositoires préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles injectables contenant des agents de dispersion et/ou des mouillants pharmacologiquement compatibles, par exemple le propylèneglycol ou le butylèneglycol.

Le principe actif peut être formulé également sous forme de micro capsules, éventuellement avec un ou plusieurs supports ou additifs, ou bien avec une matrice polymère ou avec une cyclodextrine (timbres transdermiques, formes à libération prolongée).

Les compositions topiques selon l'invention comprennent un milieu compatible avec la peau. Elles peuvent se présenter notamment sous forme de solutions aqueuses, alcooliques ou hydroalcooliques, de gels, d'émulsions eau-dans-huile ou huile-dans-eau ayant l'aspect d'une crème ou d'un gel, de micro émulsions, d'aérosols, ou encore sous forme de dispersions vésiculaires contenant des lipides ioniques et/ou non ioniques. Ces formes galéniques sont préparées selon les méthodes usuelles des domaines considérés.

## Revendications

1. Composé répondant à la formule générale (I) 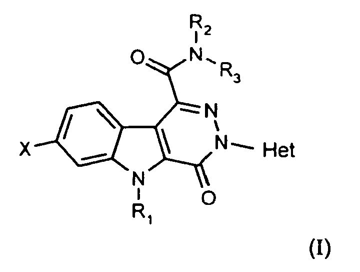 pouvant exister à l'état de base, de sel d'addition à des acides, de solvate ou d'hydrate,
dans laquelle
X représente un atome d'hydrogène ou d'halogène,
R₁ représente un atome d'hydrogène ou un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle, morpholinyle ou 4-alkylpipérazinyle, et
Het représente un groupe hétéroaromatique de type pyridinyle, 1-oxydopyridinyle, quinolinyle, isoquinolinyle, pyrimidinyle, pyrazinyle, pyridazinyle, le groupe hétéroaromatique pouvant porter un ou plusieurs atomes d'halogène et/ou un ou plusieurs groupes (C₁-C₄)alkyle, (C₁-C₄)alcoxyle.

2. Composé selon la revendication 1, **caractérisé en ce que**
X représente un atome d'halogène ;
R₁ représente un (C₁-C₄)alkyle ;
R₂ et R₃ représentent chacun, indépendamment l'un de l'autre, un atome d'hydrogène, un groupe (C₁-C₄)alkyle, ou bien R₂ et R₃ forment, avec l'atome d'azote qui les porte, un groupe pyrrolidinyle, pipéridinyle, morpholinyle ou 4-alkylpipérazinyle ;
Het représente un groupe hétéroaromatique de type pyridinyle, quinolinyle, isoquinolinyle, pyrimidinyle, pyrazinyle, pyridazinyle, pouvant porter un ou plusieurs atomes d'halogène, et/ou un ou plusieurs groupes (C₁-C₄)alkyle, (C₁-C₄)alcoxyle.

3. Composé selon la revendication 2, **caractérisé en ce que**
X représente un atome d'halogène ;
R₁ représente un (C₁-C₄)alkyle ;
R₂ et R₃ représentent chacun, indépendamment l'un de l'autre, un atome d'hydrogène, un méthyle ou un éthyle, ou bien R₂ et R₃ forment, avec l'atome d'azote qui les porte, un groupe pyrrolidinyle, pipéridinyle, morpholinyle ou 4-alkylpipérazinyle ;
Het représente un groupe hétéroaromatique de type pyridinyle, quinolinyle, isoquinolinyle, pyrimidinyle, pyrazinyle, pyridazinyle, pouvant porter un ou plusieurs atomes de brome, et/ou un ou plusieurs groupes méthyle, méthoxyle.

4. Composé selon l'une quelconque des revendications 1 à 3, **caractérisé en ce que**
X représente un atome de chlore ;
R₁ représente un groupe méthyle.

5. Composé selon la revendication 1 choisi parmi les composés suivants :
7-fluoro-*N,N*,5-triméthyl-4-oxo-3-(quinolin-3-yl)-3,5-dihydro-4*H-*pyridazino[4,5-*b*]indole-1-carboxamide
1-[[7-fluoro-5-méthyl-3-(pyridin-3-yl)-4-oxo-3,5-dihydro-4*H*-pyridazino[4,5-*b*]indol-1-yl]carbonyl]pyrrolidine
7-chloro-*N,N*,5-triméthyl-4-oxo-3-(2-méthoxypyridin-5-yl)-3,5-dihydro-4*H-*pyridazino[4,5-*b*]indole-1-carboxamide
7-chloro-*N,N*,5-triméthyl-4-oxo-3-(2-bromopyridin-5-yl)-3,5-dihydro-4*H-*pyridazino[4,5-*b*]indole-1-carboxamide
7-chloro-*N,N*,5-triméthyl-4-oxo-3-(isoquinolin-4-yl)-3,5-dihydro-4*H-*pyridazino[4,5-*b*]indole-1-carboxamide
7-chloro-*N,N*,5-triméthyl-4-oxo-3-(pyrazin-2-yl)-3,5-dihydro-4*H-*pyridazino[4,5-*b*]indole-1-carboxamide
1-[[7-chloro-5-méthyl-3-(pyridin-4-yl)-4-oxo-3,5-dihydro-4*H*-pyridazino[4,5-*b*]indol-1-yl]carbonyl]pipéridine
7-chloro-*N,N*,5-triméthyl-4-oxo-3-(2-méthylpyridin-4-yl)-3,5-dihydro-4*H-*pyridazino[4,5-*b*]indole-1-carboxamide
1-[[7-chloro-5-méthyl-3-(2-méthylpyridin-4-yl)-4-oxo-3,5-dihydro-4*H-*pyridazino[4,5-*b*]indol-1-yl]carbonyl]pyrrolidine
7-chloro-3-(2-méthoxypyridin-4-yl)-*N,N*,5-triméthyl-4-oxo-3,5-dihydro-4*H-*pyridazino[4,5-*b*]indole-1-carboxamide.

6. Composé selon la revendication 1, ce composé étant le 7-chloro-*N,N*,5-triméthyl-4-oxo-3-(2-méthoxypyridin-5-yl)-3,5-dihydro-4*H*-pyridazino[4,5-*b*]indole-1-carboxamide.

7. Procédé de préparation d'un composé de formule générale (I), 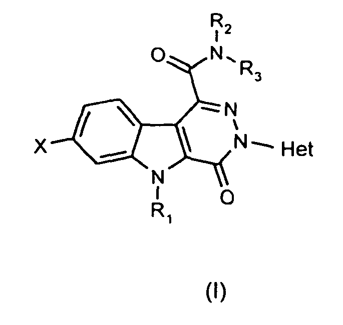 dans laquelle
X représente un atome d'hydrogène ou d'halogène,
R₁ représente un atome d'hydrogène ou un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle, morpholinyle ou 4-alkylpipérazinyle, et
Het représente un groupe hétéroaromatique de type pyridinyle, 1-oxydopyridinyle, quinolinyle, isoquinolinyle, pyrimidinyle, pyrazinyle, pyridazinyle, le groupe hétéroaromatique pouvant porter un ou plusieurs atomes d'halogène et/ou un ou plusieurs groupes (C₁-C₄)alkyle, (C₁-C₄)alcoxyle,
comprenant l'étape consistant à
transformer l'ester de formule générale (III) 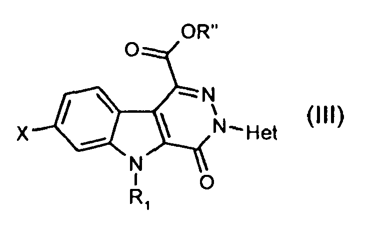 dans laquelle
X, R₁ et Het sont tels que définis ci-dessus,
R" représente un groupe (C₁-C₄)alkyle,
en amide de formule générale (I) ci-dessus,
soit par l'action d'une amine de formule générale HNR₂R₃, dans laquelle R₂ et R₃ sont tels que définis ci-dessus,
soit par saponification de l'ester de formule générale (III) en acide, puis par couplage de l'acide obtenu avec une amine de formule générale HNR₂R₃, dans laquelle R₂ et R₃ sont tels que définis ci-dessus.

8. Procédé selon la revendication 7, **caractérisé en ce que** l'ester de formule générale (III), 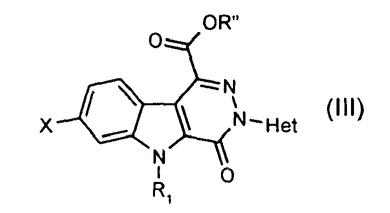 dans laquelle
R₁, Het et R" sont tels que définis **dans la revendication 7,**
est préparé en faisant réagir un composé de formule générale (II), 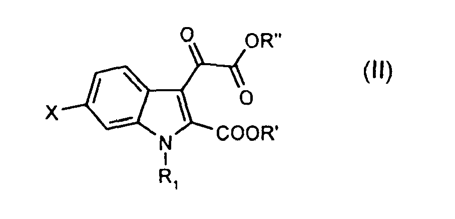 dans laquelle
X et R₁ sont tels que définis **dans la revendication 7,**
R' et R" représentent chacun indépendamment l'un de l'autre, un groupe (C₁-C₄)alkyle,
avec une hétéroarylhydrazine dans un solvant polaire en présence d'acide, à la température de reflux.

9. Procédé de préparation d'un composé de formule générale (I) 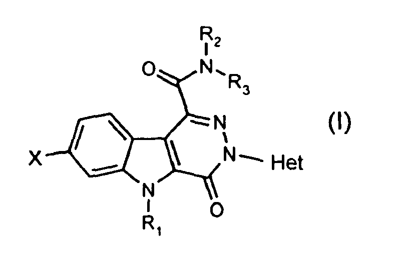 dans laquelle
X représente un atome d'hydrogène ou d'halogène,
R₁ représente un atome d'hydrogène ou un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle, morpholinyle ou 4-alkylpipérazinyle, et
Het représente un groupe hétéroaromatique de type pyridinyle, 1-oxydopyridinyle, quinolinyle, isoquinolinyle, pyrimidinyle, pyrazinyle, pyridazinyle, le groupe hétéroaromatique pouvant porter un ou plusieurs atomes d'halogène et/ou un ou plusieurs groupes (C₁-C₄)alkyle, (C₁-C₄)alcoxyle,
comprenant l'étape consistant à
réaliser une réaction de N-hétéroarylation sur une amide de formule générale (V), 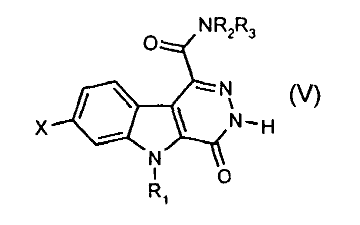 dans laquelle X, R₁, R₂ et R₃ sont tels que définis ci-dessus.

10. Procédé selon la revendication 9, **caractérisé en ce que** l'amide de formule générale (V) 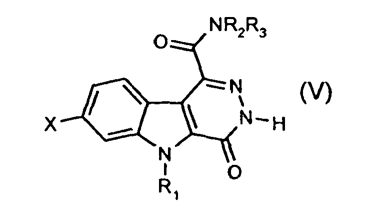 dans laquelle X, R₁, R₂ et R₃ sont tels que définis **dans la revendication 9,** est préparé en transformant l'ester de formule générale (IV) 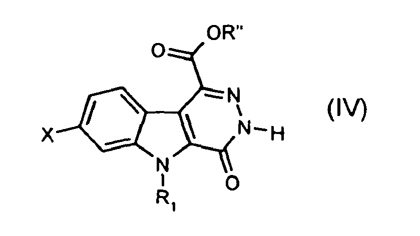 dans laquelle
X et R₁ sont tels que définis **dans la revendication 9 et R" représente un groupe (C**_{**1**}**-C**_{**4**}**)alkyle**,
soit par l'action d'une amine de formule générale HNR₂R₃, dans laquelle R₂ et R₃ sont tels que définis dans la revendication 9,
soit par saponification de l'ester de formule générale (IV) en acide, puis par couplage de l'acide obtenu avec une amine de formule générale HNR₂R₃, dans laquelle R₂ et R₃ sont tels que définis dans la revendication 9.

11. Composé répondant à la formule générale (II) 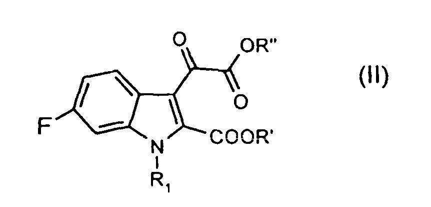 dans laquelle
R₁ représente un atome d'hydrogène ou un groupe (C₁-C₄)alkyle,
R' et R" représentent chacun, indépendamment l'un de l'autre, un groupe (Ci-C₄)alkyle.

12. Composé répondant à la formule générale (III) 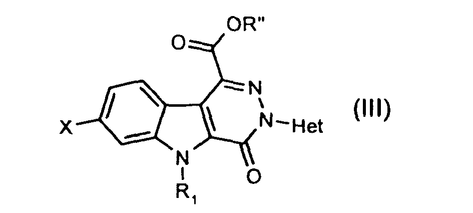 dans laquelle
X représente un atome d'hydrogène ou d'halogène,
R₁ représente un atome d'hydrogène ou un groupe (C₁-C₄)alkyle,
Het représente un groupe hétéroaromatique de type pyridinyle, 1-oxydopyridinyle, quinolinyle, isoquinolinyle, pyrimidinyle, pyrazinyle, pyridazinyle, le groupe hétéroaromatique pouvant porter un ou plusieurs atomes d'halogène et/ou un ou plusieurs groupes (C₁-C₄)alkyle, (C₁-C₄)alcoxyle,
R" représente un groupe (C₁-C₄)alkyle.

13. Composé répondant à la formule générale ci-dessous 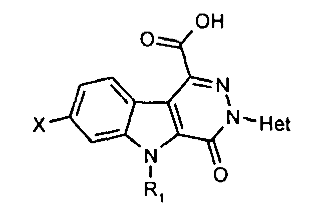 dans laquelle
X représente un atome d'hydrogène ou d'halogène,
R₁ représente un atome d'hydrogène ou un groupe (C₁-C₄)alkyle,
Het représente un groupe hétéroaromatique de type pyridinyle, 1-oxydopyridinyle, quinolinyle, isoquinolinyle, pyrimidinyle, pyrazinyle, pyridazinyle, le groupe hétéroaromatique pouvant porter un ou plusieurs atomes d'halogène et/ou un ou plusieurs groupes (C₁-C₄)alkyle, (C₁-C₄)alcoxyle,

14. Composé répondant à la formule générale (IV) 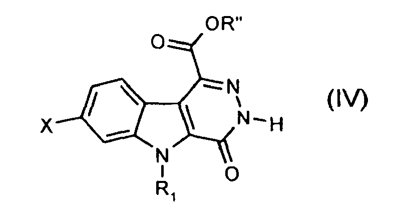 dans laquelle
X représente un atome d'hydrogène ou d'halogène,
R₁ représente un atome d'hydrogène ou un groupe (C₁-C₄)alkyle,
R" représente un groupe (C₁-C₄)alkyle.

15. Composé répondant à la formule générale ci-dessous 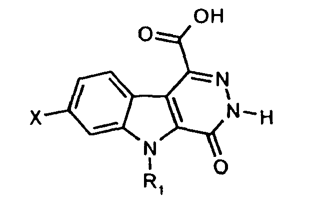 dans laquelle
X représente un atome d'hydrogène ou d'halogène,
R₁ représente un atome d'hydrogène ou un groupe (C₁-C₄)alkyle,

16. Composé répondant à la formule générale (V) 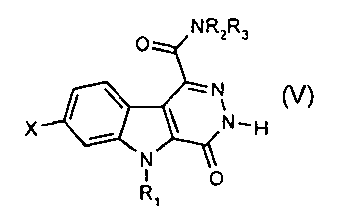 dans laquelle
X représente un atome d'hydrogène ou d'halogène,
R₁ représente un atome d'hydrogène ou un groupe (C₁-C₄)alkyle,
R₂ et R₃ représentent chacun, indépendamment l'un de l'autre, un atome d'hydrogène ou un groupe (C₁-C₄)alkyle, ou bien R₂ et R₃ forment, avec l'atome d'azote qui les porte, un groupe pyrrolidinyle, pipéridinyle, morpholinyle ou 4-alkylpipérazinyle.

17. Médicament **caractérisé en ce qu'**il consiste en un composé selon l'une quelconque des revendications **1 à 6**.

18. Composition pharmaceutique **caractérisée en ce qu'**elle contient au moins un composé selon l'une quelconque des revendications **1 à 6**, à l'état de base, de sel, de solvate ou d'hydrate pharmaceutiquement acceptables, éventuellement associé à un ou plusieurs excipients.

19. Utilisation d'un composé de formule (I) selon l'une quelconque des revendications **1 à 6** pour la préparation d'une médicament destiné à prévenir ou à traiter les neuropathies périphériques, les affections du motoneurone, les maladies neurodégénératives du système nerveux central, l'anxiété, l'épilepsie, les troubles du sommeil, l'insuffisance rénale aiguë ou chronique, la glomérulonéphrite, la néphropathie diabétique, l'ischémie et l'insuffisance cardiaques, l'infarctus du myocarde, l'ischémie des membres inférieurs, la vasospasme coronaire, l'angine de poitrine, les pathologies associées aux valves cardiaques, les maladies cardiaques inflammatoires, les effets secondaires dus à des médicaments cardiotoxiques ou aux suites d'une chirurgie cardiaque, l'athérosclérose et ses complications thrombo-emboliques, la resténose, les rejets de greffe, les conditions liées à une prolifération ou une migration incorrectes des cellules musculaires lisses, les tumeurs et cancers, les stress cutanés, les maladies inflammatoires chroniques et les maladies inflammatoires pulmonaires.

20. Utilisation d'un composé de formule (I) selon la revendication 18 où les neuropathies périphériques sont choisies parmi les neuropathies traumatiques ou ischémiques, les neuropathies infectieuses, alcooliques, médicamenteuses ou génétiques.

21. Utilisation d'un composé de formule (I) selon la revendication 18 où les affections du motoneurone sont choisies parmi les amyotrophies spinales et la sclérose latérale amyotrophique.

## Claims

1. Compound corresponding to the general formula (I) 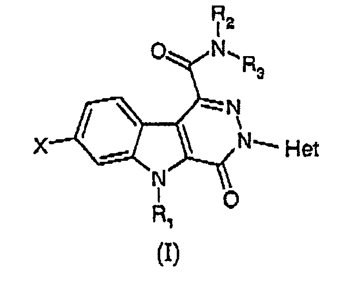 which can exist in the form of the base, of an addition salt with acids, of a solvate or of a hydrate,
in which
X represents a hydrogen or halogen atom,
R₁ represents a hydrogen atom or a (C₁-C₄)alkyl group,
R₂ and R₃ each represent, independently of one another, a hydrogen atom or a (C₁-C₄)alkyl group or else R₂ and R₃ form, with the nitrogen atom which carries them, a pyrrolidinyl, piperidinyl, morpholinyl or 4-alkylpiperazinyl group, and
Het represents a heteroaromatic group of pyridinyl, 1-oxidopyridinyl, quinolinyl, isoquinolinyl, pyrimidinyl, pyrazinyl or pyridazinyl type, where the heteroaromatic group can carry one or more halogen atoms and/or one or more (C₁-C₄)alkyl or (C₁-C₄)alkoxy groups.

2. Compound according to Claim 1, **characterized in that**
X represents a halogen atom;
R₁ represents a (C₁-C₄)alkyl;
R₂ and R₃ each represent, independently of one another, a hydrogen atom or a (C₁-C₄)alkyl group, or else R₂ and R₃ form, with the nitrogen atom which carries them, a pyrrolidinyl, piperidinyl, morpholinyl or 4-alkylpiperazinyl group;
Het represents a heteroaromatic group of pyridinyl, quinolinyl, isoquinolinyl, pyrimidinyl, pyrazinyl or pyridazinyl type, which can carry one or more halogen atoms and/or one or more (C₁-C₄)alkyl or (C₁-C₄)alkoxy groups.

3. Compound according to Claim 2,
**characterized in that**
X represents a halogen atom;
R₁ represents a (C₁-C₄)alkyl;
R₂ and R₃ each represent independently of one another, a hydrogen atom, a methyl or an ethyl, or else R₂ and R₃ form, with the nitrogen atom which carries them, a pyrrolidinyl, piperidinyl, morpholinyl or 4-alkylpiperazinyl group;
Het represents a heteroaromatic group of pyridinyl, quinolinyl, isoquinolinyl, pyrimidinyl, pyrazinyl or pyridazinyl type, which can carry one or more bromine atoms and/or one or more methyl or methoxy groups.

4. Compound according to any one of Claims 1 to 3,
**characterized in that**
X represents a chlorine atom;
R₁ represents a methyl group.

5. Compound according to Claim 1, chosen from the following compounds:
7-fluoro-N,N,5-trimethyl-4-oxo-3-(quinolin-3-yl)-3,5-dihydro-4H-pyridazino[4,5-*b*]indole-1-carboxamide, 1-[[7-fluoro-5-methyl-3-(pyridin-3-yl)-4-oxo-3,5-dihydro-4*H*-pyridazino[4,5-*b*]indol-1-yl]carbonyl]pyrrolidine, 7-chloro-*N,N*,5-trimethyl-4-oxo-3-(2-methoxypyridin-5-yl)-3,5-dihydro-4*H*-pyridazino[4,5-*b*]indole-1-carboxamide, 7-chloro-*N,N*,5-trimethyl-4-oxo-3-(2-bromopyridin-5-yl)-3,5-dihydro-4*H*-pyridazino[4,5-*b*]indole-1-carboxamide, 7-chloro-*N,N*,5-trimethyl-4-oxo-3-(isoquinolin-4-yl)-3,5-dihydro-4*H*-pyridazino[4,5-*b*]indole-1-carboxamide, 7-chloro-*N,N*,5-trimethyl-4-oxo-3-(pyrazin-2-yl)-3,5-dihydro-4*H-*pyridazino[4,5-*b*]indole-1-carboxamide, 1-[[7-chloro-5-methyl-3-(pyridin-4-yl)-4-oxo-3,5-dihydro-4*H*-pyridazino[4,5-*b*]indol-1-yl]carbonyl]piperidine, 7-chloro-*N,N*,5-trimethyl-4-oxo-3-(2-methylpyridin-4-yl)-3,5-dihydro-4*H*-pyridazino[4,5-*b*]indole-1-carboxamide, 1-[[7-chloro-5-methyl-3-(2-methylpyridin-4-yl)-4-oxo-3,5-dihydro-4*H-*pyridazino[4,5-*b*]indol-1-yl]carbonyl]pyrrolidine, 7-chloro-3-(2-methoxypyridin-4-yl)-*N,N*,5-trimethyl-4-oxo-3,5-dihydro-4*H*-pyridazino[4,5-*b*]indole-1-carboxamide.

6. Compound according to Claim 1, this compound being 7-chloro-*N,N*,5-trimethyl-4-oxo-3-(2-methoxypyridin-5-yl)-3,5-dihydro-4*H*-pyridazino[4,5-*b*]indole-1-carboxamide.

7. Process for the preparation of a compound of general formula (I), 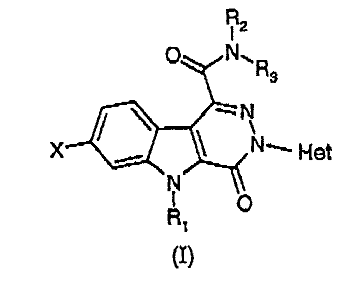 in which
X represents a hydrogen or halogen atom,
R₁ represents a hydrogen atom or a (C₁-C₄)alkyl group,
R₂ and R₃ each represent, independently of one another, a hydrogen atom or a (C₁-C₄)alkyl group or else R₂ and R₃ form, with the nitrogen atom which carries them, a pyrrolidinyl, piperidinyl, morpholinyl or 4-alkylpiperazinyl group, and
Het represents a heteroaromatic group of pyridinyl, 1-oxidopyridinyl, quinolinyl, isoquinolinyl, pyrimidinyl, pyrazinyl or pyridazinyl type, where the heteroaromatic group can carry one or more halogen atoms and/or one or more (C₁-C₄)alkyl or (C₁-C₄)alkoxy groups, comprising the stage consisting in converting the ester of general formula (III) 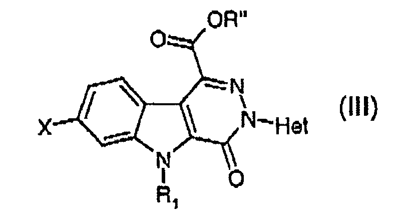 in which
X, R₁ and Het are as defined above,
R" represents a (C₁-C₄)alkyl group,
to the amide of general formula (I) above,
either by reaction with an amine of general formula HNR₂R₃, in which R₂ and R₃ are as defined above,
or by saponification of the ester of general formula (III) to the acid and then by coupling the acid obtained with an amine of general formula HNR₂R₃, in which R₂ and R₃ are as defined above.

8. Process according to Claim 7, **characterized in that** the ester of general formula (III), 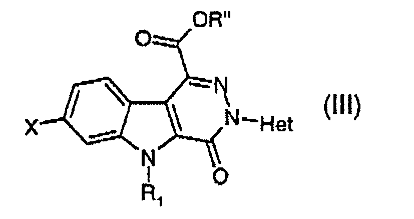 in which
R₁, Het and R" are as defined in Claim 7,
is prepared by reacting a compound of general formula (II), 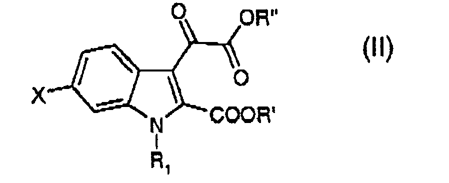 in which
X and R₁ are as defined in Claim 7,
R' and R" each represent, independently of one another, a (C₁-C₄)alkyl group,
with a heteroarylhydrazine in a polar solvent in the presence of acid at the reflux temperature.

9. Process for the preparation of a compound of general formula (I) 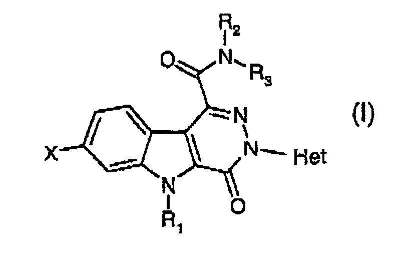 in which
X represents a hydrogen or halogen atom,
R₁ represents a hydrogen atom or a (C₁-C₄)alkyl group,
R₂ and R₃ each represent, independently of one another, a hydrogen atom or a (C₁-C₄)alkyl group or else R₂ and R₃ form, with the nitrogen atom which carries them, a pyrrolidinyl, piperidinyl, morpholinyl or 4-alkylpiperazinyl group, and
Het represents a heteroaromatic group of pyridinyl, 1-oxidopyridinyl, quinolinyl, isoquinolinyl, pyrimidinyl, pyrazinyl or pyridazinyl type, where the heteroaromatic group can carry one or more halogen atoms and/or one or more (C₁-C₄)alkyl or (C₁-C₄)alkoxy groups, comprising the stage consisting in
carrying out an N-heteroarylation reaction on an amide of general formula (V), 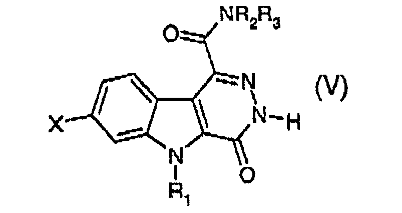 in which X, R₁, R₂ and R₃ are as defined above.

10. Process according to Claim 9, **characterized in that** the amide of general formula (V) 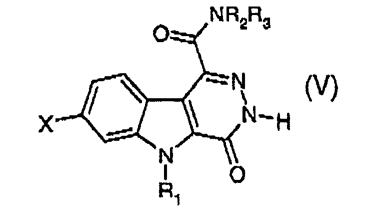 in which X, R₁, R₂ and R₃ are as defined in Claim 9,
is prepared by converting the ester of general formula (IV) 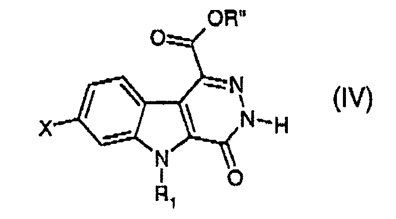 in which
X and R₁ are as defined in Claim 9 and R" represents a (C₁-C₄)alkyl group,
either by reaction with an amine of general formula HNR₂R₃, in which R₂ and R₃ are as defined in Claim 9,
or by saponification of the ester of general formula (IV) to the acid and then by coupling the acid obtained with an amine of general formula HNR₂R₃, in which R₂ and R₃ are as defined in Claim 9.

11. Compound corresponding to the general formula (II) 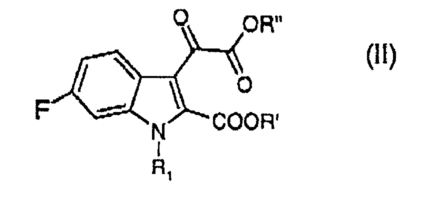 in which
R₁ represents a hydrogen atom or a (C₁-C₄)alkyl group,
R' and R" each represent, independently of one another, a (C₁-C₄)alkyl group.

12. Compound corresponding to the general formula (III) 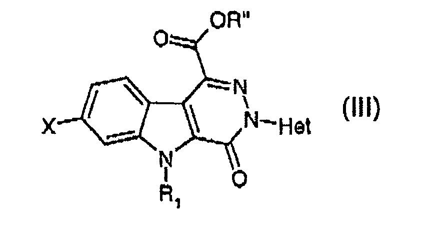 in which
X represents a hydrogen or halogen atom,
R₁ represents a hydrogen atom or a (C₁-C₄)alkyl group,
Het represents a heteroaromatic group of pyridinyl, 1-oxidopyridinyl, quinolinyl, isoquinolinyl, pyrimidinyl, pyrazinyl or pyridazinyl type, where the heteroaromatic group can carry one or more halogen atoms and/or one or more (C₁-C₄)alkyl or (C₁-C₄)alkoxy groups,
R" represents a (C₁-C₄)alkyl group.

13. Compound corresponding to the general formula below 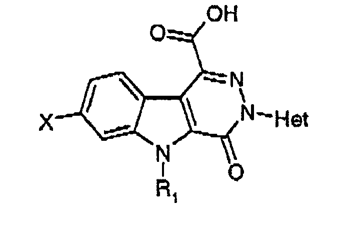 in which
X represents a hydrogen or halogen atom,
R₁ represents a hydrogen atom or a (C₁-C₄)alkyl group,
Het represents a heteroaromatic group of pyridinyl, 1-oxidopyridinyl, quinolinyl, isoquinolinyl, pyrimidinyl, pyrazinyl or pyridazinyl type, where the heteroaromatic group can carry one or more halogen atoms and/or one or more (C₁-C₄)alkyl or (C₁-C₄)alkoxy groups.

14. Compound corresponding to the general formula (IV) 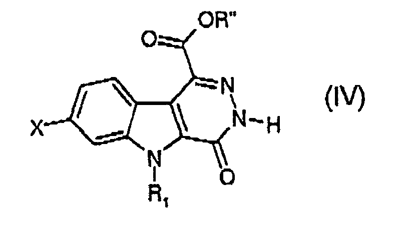 in which
X represents a hydrogen or halogen atom,
R₁ represents a hydrogen atom or a (C₁-C₄)alkyl group,
R" represents a (C₁-C₄)alkyl group.

15. Compound corresponding to the general formula below 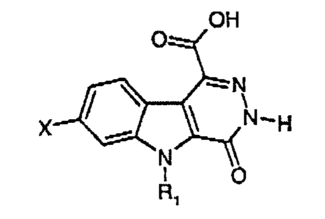 in which
X represents a hydrogen or halogen atom,
R₁ represents a hydrogen atom or a (C₁-C₄)alkyl group.

16. Compound corresponding to the general formula (V) 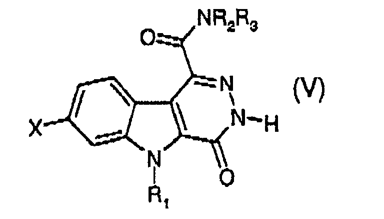 in which
X represents a hydrogen or halogen atom,
R₁ represents a hydrogen atom or a (C₁-C₄)alkyl group,
R₂ and R₃ each represent, independently of one another, a hydrogen atom or a (C₁-C₄)alkyl group or else R₂ and R₃ form, with the nitrogen atom which carries them, a pyrrolidinyl, piperidinyl, morpholinyl or 4-alkylpiperazinyl group.

17. Medicament, **characterized in that** it comprises a compound according to any one of Claims 1 to 6.

18. Pharmaceutical composition, **characterized in that** it comprises at least one compound according to any one of Claims 1 to 6, in the form of the base, of a pharmaceutically acceptable salt, of a pharmaceutically acceptable solvate or of a pharmaceutically acceptable hydrate, optionally in combination with one or more excipients.

19. Use of a compound of formula (I) according to any one of Claims 1 to 6 for the preparation of a medicament intended to prevent or treat peripheral neuropathies, motor neurone conditions, neurodegenerative diseases of the central nervous system, anxiety, epilepsy, sleep disorders, acute or chronic renal insufficiency, glomerulonephritis, diabetic nephropathy, cardiac ischaemia and cardiac insufficiency, myocardial infarction, ischaemia of the lower limbs, coronary vasospasm, angina pectoris, pathologies associated with the heart valves, inflammatory heart diseases, side effects due to cardiotoxic medicaments or as a result of heart surgery, atherosclerosis and its thromboembolic complications, restenosis, graft rejections, conditions related to incorrect proliferation or incorrect migration of smooth muscle cells, tumours and cancers, cutaneous stress, chronic inflammatory diseases and pulmonary inflammatory diseases.

20. Use of a compound of formula (I) according to Claim 18, where the peripheral neuropathies are chosen from traumatic or ischaemic neuropathies or infections, alcoholic, medicinal or genetic neuropathies.

21. Use of a compound of formula (I) according to Claim 18, where the motor neurone conditions are chosen form spinal amyotrophies and amyotrophic lateral sclerosis.

## Patentansprüche

1. Verbindung der allgemeinen Formel (I) 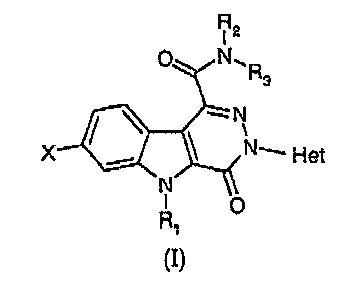 die in Basen-, Säureadditionssalz-, Solvat- oder Hydratform vorliegen kann,
worin
X für ein Wasserstoff- oder Halogenatom steht,
R₁ für ein Wasserstoffatom oder eine (C₁-C₄)-Alkylgruppe steht,
R₂ und R₃ jeweils unabhängig voneinander für ein Wasserstoffatom oder eine (C₁-C₄)-Alkylgruppe stehen oder mit dem Stickstoffatom, an das sie gebunden sind, eine Pyrrolidinyl-, Piperidinyl-, Morpholinyl- oder 4-Alkylpiperazinylgruppe bilden und
Het für eine heteroaromatische Gruppe vom Pyridinyl-, 1-Oxidopyridinyl-, Chinolinyl-, Isochinolinyl-, Pyrimidinyl-, Pyrazinyl- oder Pyridazinyltyp steht, wobei die heteroaromatische Gruppe ein oder mehrere Halogenatome und/oder eine oder mehrere (C₁-C₄)-Alkyl- oder (C₁-C₄)-Alkoxygruppen tragen kann.

2. Verbindung nach Anspruch 1, **dadurch gekennzeichnet, daß**
X für ein Halogenatom steht,
R₁ für eine (C₁-C₄)-Alkylgruppe steht,
R₂ und R₃ jeweils unabhängig voneinander für ein Wasserstoffatom oder eine (C₁-C₄)-Alkylgruppe stehen oder mit dem Stickstoffatom, an das sie gebunden sind, eine Pyrrolidinyl-, Piperidinyl-, Morpholinyl- oder 4-Alkylpiperazinylgruppe bilden und
Het für eine heteroaromatische Gruppe vom Pyridinyl-, Chinolinyl-, Isochinolinyl-, Pyrimidinyl-, Pyrazinyl- oder Pyridazinyltyp steht, die ein oder mehrere Halogenatome und/oder eine oder mehrere (C₁-C₄)-Alkyl- oder (C₁-C₄)-Alkoxygruppen tragen kann.

3. Verbindung nach Anspruch 2, **dadurch gekennzeichnet, daß**
X für ein Halogenatom steht,
R₁ für eine (C₁-C₄)-Alkylgruppe steht,
R₂ und R₃ jeweils unabhängig voneinander für ein Wasserstoffatom, Methyl oder Ethyl stehen oder mit dem Stickstoffatom, an das sie gebunden sind, eine Pyrrolidinyl-, Piperidinyl-, Morpholinyl- oder 4-Alkylpiperazinylgruppe bilden und
Het für eine heteroaromatische Gruppe vom Pyridinyl-, Chinolinyl-, Isochinolinyl-, Pyrimidinyl-, Pyrazinyl- oder Pyridazinyltyp steht, die ein oder mehrere Bromatome und/oder eine oder mehrere Methyl- oder Methoxygruppen tragen kann.

4. Verbindung nach einem der Ansprüche 1 bis 3,
**dadurch gekennzeichnet, daß**
X für ein Chloratom steht und
R₁ für eine Methylgruppe steht.

5. Verbindung nach Anspruch 1, ausgewählt unter den folgenden Verbindungen:
7-Fluor-*N,N*,5-trimethyl-4-oxo-3-(chinolin-3-yl)-3,5-dihydro-4*H*-pyridazino[4,5-*b*]indol-1-carboxamid 1-[[7-Fluor-5-methyl-3-(pyridin-3-yl)-4-oxo-3,5-dihydro-4*H*-pyridazino[4,5-*b*]indol-1-yl]-carbonyl]pyrrolidin
7-Chlor-*N,N*,5-trimethyl-4-oxo-3-(2-methoxypyridin-5-yl)-3,5-dihydro-4*H*-pyridazino[4,5-*b*]indol-1-carboxamid
7-Chlor-*N,N*,5-trimethyl-4-oxo-3-(2-brompyridin-5-yl)-3,5-dihydro-4*H*-pyridazino[4,5-*b*]indol-1-carboxamid
7-Chlor-*N,N*,5-trimethyl-4-oxo-3-(isochinolin-4-yl)-3,5-dihydro-4*H*-pyridazino[4,5-*b*]indol-1-carboxamid
7-Chlor-*N,N*,5-trimethyl-4-oxo-3-(pyrazin-2-yl)-3,5-dihydro-4*H*-pyridazino[4,5-*b*]indol-1-carboxamid 1-[[7-Chlor-5-methyl-3-(pyridin-4-yl)-4-oxo-3,5-dihydro-4*H*-pyridazino[4,5-*b*]indol-1-yl]-carbonyl]piperidin
7-Chlor-*N,N*,5-trimethyl-4-oxo-3-(2-methylpyridin-4-yl)-3,5-dihydro-4*H*-pyridazino[4,5-*b*]indol-1-carboxamid
1-[[7-Chlor-5-methyl-3-(2-methylpyridin-4-yl)-4-oxo-3,5-dihydro-4*H*-pyridazino[4,5-*b*]indol-1-yl]-carbonyl]pyrrolidin
7-Chlor-3-(2-methoxypyridin-4-yl)-*N,N*,5-trimethyl-4-oxo-3,5-dihydro-4*H*-pyridazino[4,5-*b*]indol-1-carboxamid.

6. Verbindung nach Anspruch 1, bei der es sich um 7-Chlor-*N*,*N*,5-trimethyl-4-oxo-3-(2-methoxypyridin-5-yl)-3,5-dihydro-4*H*-pyridazino[4,5-*b*]indol-1-carboxamid handelt.

7. Verfahren zur Herstellung einer Verbindung der allgemeinen Formel (I) 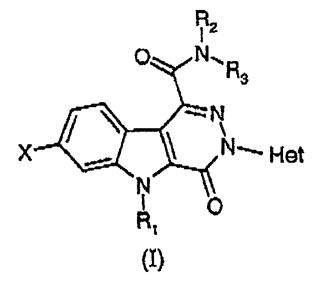 worin
X für ein Wasserstoff- oder Halogenatom steht,
R₁ für ein Wasserstoffatom oder eine (C₁-C₄)-Alkylgruppe steht,
R₂ und R₃ jeweils unabhängig voneinander für ein Wasserstoffatom oder eine (C₁-C₄)-Alkylgruppe stehen oder mit dem Stickstoffatom, an das sie gebunden sind, eine Pyrrolidinyl-, Piperidinyl-, Morpholinyl- oder 4-Alkylpiperazinylgruppe bilden und
Het für eine heteroaromatische Gruppe vom Pyridinyl-, 1-Oxidopyridinyl-, Chinolinyl-, Isochinolinyl-, Pyrimidinyl-, Pyrazinyl- oder Pyridazinyltyp steht, wobei die heteroaromatische Gruppe ein oder mehrere Halogenatome und/oder eine oder mehrere (C₁-C₄)-Alkyl- oder (C₁-C₄)-Alkoxygruppen tragen kann,
bei dem man
den Ester der allgemeinen Formel (III) 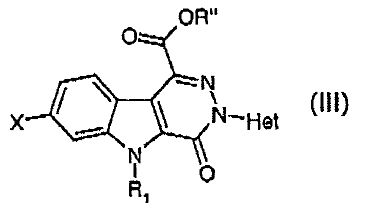 worin
X, R₁ und Het die oben angegebene Bedeutung besitzen,
R für eine (C₁-C₄)-Alkylgruppe steht,
entweder durch Einwirkung eines Amins der allgemeinen Formel HNR₂R₃, worin R₂ und R₃ die oben angegebene Bedeutung besitzen,
oder durch Verseifung des Esters der allgemeinen Formel (III) zur Säure und nachfolgende Kupplung der erhaltenen Säure mit einem Amin der allgemeinen Formel HNR₂R₃, worin R₂ und R₃ die oben angegebene Bedeutung besitzen, in das Amid der obigen allgemeinen Formel (I) umwandelt.

8. Verfahren nach Anspruch 7, **dadurch gekennzeichnet, daß** man den Ester der allgemeinen Formel (III) 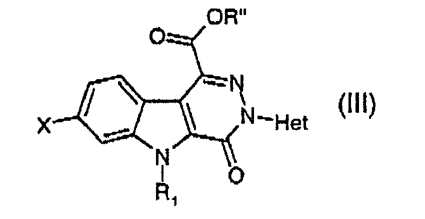 worin
R₁, Het und R" die in Anspruch 7 angegebene Bedeutung besitzen,
durch Umsetzung einer Verbindung der allgemeinen Formel (II) 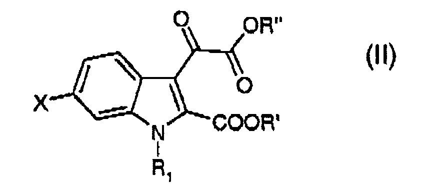 worin
X und R₁ die in Anspruch 7 angegebene Bedeutung besitzen und
R' und R" jeweils unabhängig voneinander für eine (C₁-C₄)-Alkylgruppe stehen,
mit einem Heteroarylhydrazin in einem polaren Lösungsmittel in Gegenwart einer Säure bei Rückflußtemperatur herstellt.

9. Verfahren zur Herstellung einer Verbindung der allgemeinen Formel (I) 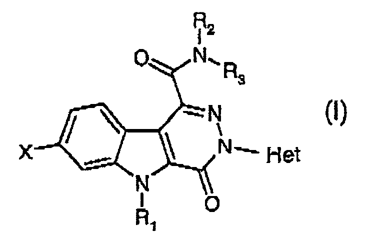 worin
X für ein Wasserstoff- oder Halogenatom steht,
R₁ für ein Wasserstoffatom oder eine (C₁-C₄)-Alkylgruppe steht,
R₂ und R₃ jeweils unabhängig voneinander für ein Wasserstoffatom oder eine (C₁-C₄)-Alkylgruppe stehen oder mit dem Stickstoffatom, an das sie gebunden sind, eine Pyrrolidinyl-, Piperidinyl-, Morpholinyl- oder 4-Alkylpiperazinylgruppe bilden und
Het für eine heteroaromatische Gruppe vom Pyridinyl-, 1-Oxidopyridinyl-, Chinolinyl-, Isochinolinyl-, Pyrimidinyl-, Pyrazinyl- oder Pyridazinyltyp steht, wobei die heteroaromatische Gruppe ein oder mehrere Halogenatome und/oder eine oder mehrere (C₁-C₄)-Alkyl- oder (C₁-C₄)-Alkoxygruppen tragen kann,
bei dem man
an einem Amid der allgemeinen Formel (V) 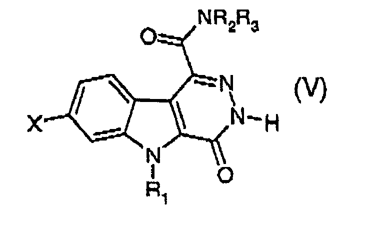 worin X, R₁, R₂ und R₃ die oben angegebene Bedeutung besitzen,
eine *N*-Heteroarylierungsreaktion durchführt.

10. Verfahren nach Anspruch 9, **dadurch gekennzeichnet, daß** man das Amid der allgemeinen Formel (V) 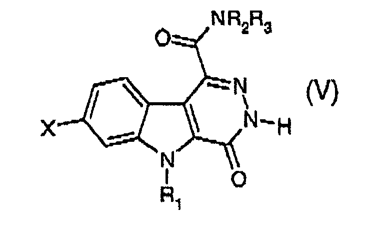 worin X, R₁, R₂ und R₃ die in Anspruch 9 angegebene Bedeutung besitzen,
durch Umwandlung des Esters der allgemeinen Formel (IV) 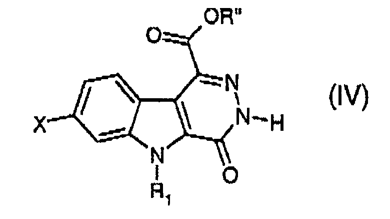 worin
X und R₁ die in Anspruch 9 angegebene Bedeutung besitzen und R" für eine (C₁-C₄)-Alkylgruppe steht,
entweder durch Einwirkung eines Amins der allgemeinen Formel HNR₂R₃, worin R₂ und R₃ die in Anspruch 9 angegebene Bedeutung besitzen,
oder durch Verseifung des Esters der allgemeinen Formel (IV) zur Säure und nachfolgende Kupplung der erhaltenen Säure mit einem Amin der allgemeinen Formel HNR₂R₃, worin R₂ und R₃ die in Anspruch 9 angegebene Bedeutung besitzen, herstellt.

11. Verbindung der allgemeinen Formel (II) 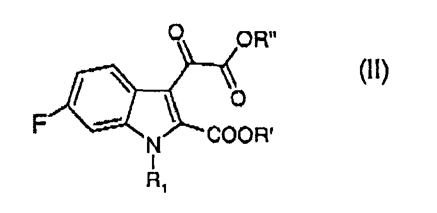 worin
R₁ für ein Wasserstoffatom oder eine (C₁-C₄)-Alkylgruppe steht und
R' und R" jeweils unabhängig voneinander für eine (C₁-C₄)-Alkylgruppe stehen.

12. Verbindung der allgemeinen Formel (III) 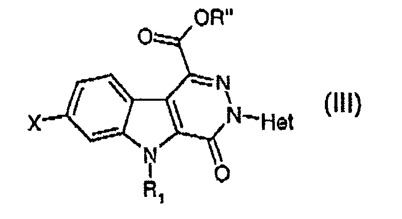 worin
X für ein Wasserstoff- oder Halogenatom steht,
R₁ für ein Wasserstoffatom oder eine (C₁-C₄)-Alkylgruppe steht,
Het für eine heteroaromatische Gruppe vom Pyridinyl-, 1-Oxidopyridinyl-, Chinolinyl-, Isochinolinyl-, Pyrimidinyl-, Pyrazinyl- oder Pyridazinyltyp steht, wobei die heteroaromatische Gruppe ein oder mehrere Halogenatome und/oder eine oder mehrere (C₁-C₄)-Alkyl- oder (C₁-C₄)-Alkoxygruppen tragen kann, und
R" für eine (C₁-C₄)-Alkylgruppe steht.

13. Verbindung der nachstehenden allgemeinen Formel 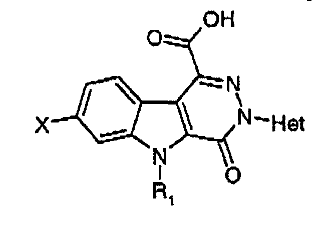 worin
X für ein Wasserstoff- oder Halogenatom steht,
R₁ für ein Wasserstoffatom oder eine (C₁-C₄)-Alkylgruppe steht und
Het für eine heteroaromatische Gruppe vom Pyridinyl-, 1-Oxidopyridinyl-, Chinolinyl-, Isochinolinyl-, Pyrimidinyl-, Pyrazinyl- oder Pyridazinyltyp steht, wobei die heteroaromatische Gruppe ein oder mehrere Halogenatome und/oder eine oder mehrere (C₁-C₄)-Alkyl- oder (C₁-C₄)-Alkoxygruppen tragen kann.

14. Verbindung der allgemeinen Formel (IV) 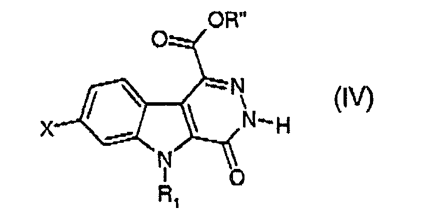 worin
X für ein Wasserstoff- oder Halogenatom steht,
R₁ für ein Wasserstoffatom oder eine (C₁-C₄)-Alkylgruppe steht und
R" für eine (C₁-C₄)-Alkylgruppe steht.

15. Verbindung der nachstehenden allgemeinen Formel 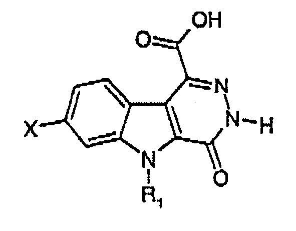 worin
X für ein Wasserstoff- oder Halogenatom steht und
R₁ für ein Wasserstoffatom oder eine (C₁-C₄)-Alkylgruppe steht.

16. Verbindung der allgemeinen Formel (V) 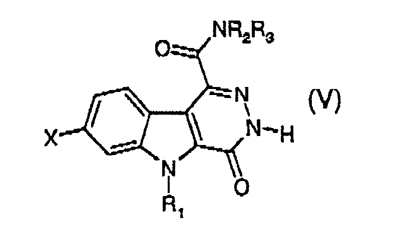 worin
X für ein Wasserstoff- oder Halogenatom steht,
R₁ für ein Wasserstoffatom oder eine (C₁-C₄)-Alkylgruppe steht und
R₂ und R₃ jeweils unabhängig voneinander für ein Wasserstoffatom oder eine (C₁-C₄)-Alkylgruppe stehen oder mit dem Stickstoffatom, an das sie gebunden sind, eine Pyrrolidinyl-, Piperidinyl-, Morpholinyl- oder 4-Alkylpiperazingruppe bilden.

17. Arzneistoff, **dadurch gekennzeichnet, daß** er aus einer Verbindung nach einem der Ansprüche 1 bis 6 besteht.

18. Pharmazeutische Zusammensetzung, **dadurch gekennzeichnet, daß** sie mindestens eine Verbindung nach einem der Ansprüche 1 bis 6 in pharmazeutisch unbedenklicher Basen-, Säureadditionssalz-, Solvat- oder Hydratform enthält, gegebenenfalls zusammen mit einem oder mehreren Trägerstoffen.

19. Verwendung einer Verbindung der Formel (I) nach einem der Ansprüche 1 bis 6 zur Herstellung eines Arzneimittels zur Prävention oder Behandlung von peripheren Neuropathien, Motoneuronenleiden, neurodegenerativen Erkrankungen des Zentralnervensystems, Angst, Epilepsie, Schlafstörungen, akuter oder chronischer Niereninsuffizienz, Glomerulonephritis, diabetischer Nephropathie, Herzischämie, Herzinsuffizienz, Myokardinfarkt, Ischämie der unteren Extremitäten, koronarem Gefäßspasmus, Angina pectoris, mit Herzklappen assoziierten Pathologien, entzündlichen Herzerkrankungen, Nebenwirkungen von kardiotoxischen Arzneimitteln oder nach Herzchirurgie, Atherosklerose und deren thromboembolischen Komplikationen, Restenose, Transplantatabstoßungen, mit inkorrekter Proliferation oder Migration von glatten Muskelzellen verbundenen Zuständen, Tumoren und Krebs, Hautstreß, chronischen entzündlichen Erkrankungen und entzündlichen Lungenerkrankungen.

20. Verwendung einer Verbindung der Formel (I) nach Anspruch 18, bei der die peripheren Neuropathien unter traumatischen oder ischämischen Neuropathien, infektiösen, alkoholischen, medikamentösen oder genetischen Neuropathien ausgewählt sind.

21. Verwendung einer Verbindung der Formel (I) nach Anspruch 18, bei der die Motoneuronenleiden unter spinalen Amyotrophien und amyotropher Lateralsklerose ausgewählt sind.
